# EUROPEAN PATENT APPLICATION

(11) **EP 0 967 212 A1**
(43) Date of publication of application: **29.12.1999**
(21) Application number: 97947954.0
(22) Date of filing: 16.12.1997
(51) Int. Cl.: C07D 401/04, C07D 403/04, A01N 43/56

(54) **PYRAZOLINE COMPOUNDS AND USE AS PLANT DISEASE CONTROL AGENT**

(30) Priority: 16.12.1996 JP 33572996
(71) Applicant: SUMITOMO CHEMICAL COMPANY LIMITED, Osaka-shi, Osaka 541-0041 (JP)
(72) Inventor: TAKI, Toshiaki, Toyonaka-shi, Osaka 561 (JP); SATO, Junichi, Toyonaka-shi, Osaka 561 (JP); KIMURA, Norio, Takarazuka-shi, Hyogo 665 (JP)
(74) Representative: VOSSIUS & PARTNER
(86) International application number: JP9704627
(87) International publication number: WO9827084

(57) **Abstract**

A pyrazoline compound represented by the formula (I): 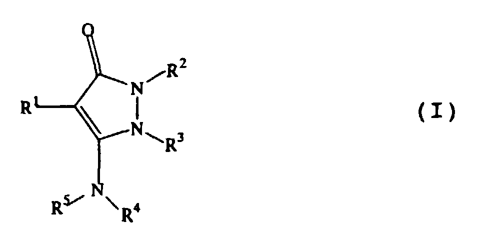 wherein R¹ represents a phenyl group which may be substituted, R² represents a hydrocarbon group which may be substituted, R³ represents an alkyl group or the like, R⁴ and R⁵, which may be the same or different, represent a hydrogen atom or the like, A and B, which may be the same or different, represents a group CH or the like, is used as an effective ingredient of plant disease controlling agent.

## Description

The present invention relates to pyrazoline compounds, their use as plant disease controlling agents, and intermediates for preparing them.

The object of the present invention is to provide a compound having an excellent controlling effect against plant disease in which the pyrazoline compound represented by the following general formula (I) has an excellent controlling effect against plant diseases. That is to say, the present invention provides the pyrazoline compounds represented by the formula (I): 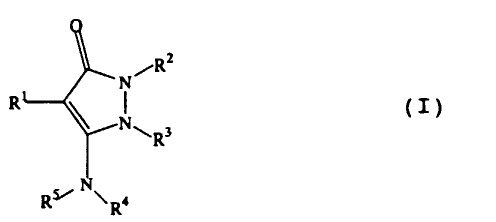 (referred to hereinafter as the present compound) wherein R¹ represents a phenyl group which may be substituted, R² represents a hydrocarbon group which may be substituted, R³ represents an aromatic heterocyclic ring which may be substituted, R⁴ and R⁵, which may be the same or different, represent a hydrogen atom, an acyl group, a primary or secondary alkyl group which may be substituted, or are combined together to form an alkylene group which may be substituted or =CR⁶R⁷, wherein R⁶ represents a hydrocarbon group which may be substituted, an alkoxy group, or a mono- or di-alkylamino group, and R⁷ represents a hydrogen atom or an alkyl group, and a plant disease controlling agent comprising the compound (I) as an effective ingredient.

The present invention also provides the aminopyrazoline compounds useful as the intermediate for preparing the present compound and represented by the formula (II): 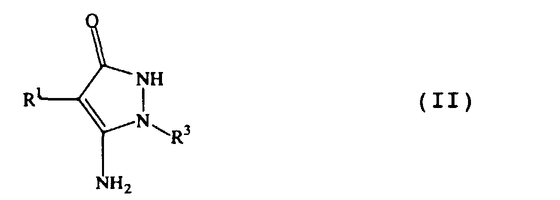 wherein R¹ and R³ have the same meanings as defined above.

The aminopyrazoline compounds represented by the formula (II) can take the following tautomeric structures

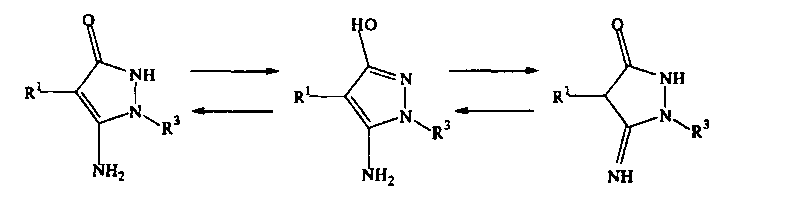

In the present invention, the substituent in the phenyl group which may be substituted for R¹ includes for example a halogen atom such as a fluorine atom, a chlorine atom, a bromine atom and an iodine atom; a C₁-C₆ alkyl group such as a methyl group, ethyl group, propyl group and isopropyl group; a C₁-C₆ haloalkyl group such as a trifluoromethyl group, a pentafluoroethyl group and a heptafluoropropyl group; a C₁-C₆ alkoxy group such as a methoxy group, an ethoxy group, a propoxy group and an isopropoxy group; a C₁-C₆ haloalkoxy group such as a trifluoromethoxy group, a difluoromethoxy group, a pentafluoroethoxy group, a heptafluoropropoxy group, a chlorodifluoromethoxy group, a bromodifluoromethoxy group and a 2,2,2-trifluoroethoxy group; a (C₁-C₆) alkoxy (C₁-C₆) alkyl group such as a methoxymethyl group; a C₁-C₆ alkylthio group such as a methylthio group and an ethylthio group; a C₁-C₆ haloalkylthio group such as a trifluoromethylthio group, a difluoromethylthio group and a 2,2,2-trifluoroethylthio group; a cyano group; a nitro group; a phenyl group; or a phenoxy group. In this connection, the phenyl and phenoxy groups may be substituted by a halogen atom such as a fluorine atom, a chlorine atom, a bromine atom and an iodine atom, a C₁-C₆ alkyl group such as a methyl group, an ethyl group, a propyl group and an isopropyl group, a C₁-C₆ haloalkyl group such as a trifluoromethyl group, a C₁-C₆ alkoxy group such as a methoxy group and an ethoxy group, a C₁-C₆ haloalkoxy group such as a trifluoromethoxy group and difluoromethoxy group, a cyano group and/or a nitro group. In addition, these substituents may be present one or more, and the substituents may be the same or different when two or more of such substituents are present. Furthermore, the substituent in the phenyl group which may be substituted for R¹ may also be two adjacent substituents represented by -CH=CH-CH=CH- or a C₁-C₅ alkylene group which forms a 3 - 7 membered cyclic ring such as a methylene group, an ethylene group, a trimethylene group, a tetramethylene group and a pentamethylene group. In this connection, the C₁-C₅ alkylene group may be substituted by a halogen atom such as a fluorine atom, a chlorine atom, a bromine atom and an iodine atom, and/or a C₁-C₃ alkyl group such as a methyl group, an ethyl group, a propyl group and an isopropyl group, and one or two oxygen or sulfur atoms may be contained in the alkylene chain.

The hydrocarbon group which may be substituted for R² includes for example C₁-C₁₀ alkyl group such as an ethyl group, a propyl group, an isopropyl group, a butyl group, an iso-butyl group, a sec-butyl group, a tert-butyl group, a 1-methylpropyl group, a 1-methylbutyl group and a 1-ethylpropyl group; a C₃-C₇ cycloalkyl group such as a cyclopentyl group and a cyclohexyl group; a C₁-C₆ haloalkyl group such as a 1-(trifluoromethyl)ethyl group; a (C₁-C₆) alkoxy (C₁-C₆) alkyl group such as a 1-methoxyethyl group and a 1-ethoxyethyl group; a C₃-C₁₀ alkenyl group such as an allyl group and a 1-methyl-2-propenyl group; a C₃-C₁₀ haloalkenyl group such as a 3,3-dichloro-2-propenyl group and a 3,3-dibromo-2-propenyl group; a C₃-C₁₀ alkynyl group such as a prorpagyl group and a 1-methyl-2-propynyl group; a C₃-C₁₀ haloalkynyl group such as a 4,4,4-trifluoro-2-butynyl group, a 4-bromo-4,4-difluoro-2-butynyl group and a 4-bromo-4,4-difluoro-2-butynyl group; or a phenyl group which may be substituted by a halogen atom such as a fluorine atom, a chlorine atom, a bromine atom and an iodine atom, a C₁-C₆ alkyl group such as a methyl group, an ethyl group, a propyl group and an isopropyl group, a C₁-C₆ haloalkyl group such as a trifluoromethyl group, a C₁-C₆ alkoxy group such as a methoxy group and an ethoxy group, a C₁-C₆ haloalkoxy group such as a trifluoromethoxy group and difluoromethoxy group, a cyano group or a nitro group.

The aromatic heterocyclic ring which may be substituted for R³ includes for example the ones represented by the formula 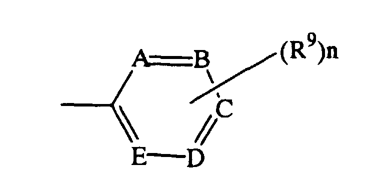 wherein R⁹ represents a C₁-C₆ alkyl group, a C₁-C₆ alkoxy group, a C₁-C₆ alkylthio group, a C₁-C₆ haloalkyl group, a C₁-C₆ haloalkoxy group, a C₁-C₆ haloalkylthio group, or a halogen atom, A, B, C, D and E, which may be the same or different, represent a CH group or a nitrogen atom, respectively. n represents an integer of 0 - 4, when either one of A - E represents a nitrogen atom, n represents an integer of 0 - 3, when two of A - E represent a nitrogen atom, n represents an integer of 0 - 2, when three of A - E represent a nitrogen atom, and n represents an integer of 0 - 1, when four of A - E represent a nitrogen atom. More particularly, the aromatic heterocyclic ring includes for example a 2-pyridyl group, a 3-pyridyl group, a 4-pyridyl group, a 2-pyrimidinyl group, a 3-pyrimidinyl group, a 4-pyrimidinyl group, a 2-pyrazinyl group, a 3-pyridazinyl group, a 4-pyridazinyl group, a 1,3,5-triazin-2-yl group, a 1,2,4-triazin-3-yl group, a 1,2,4-triazin-5-yl group, a 1,2,4-triazin-6-yl group, a 1,2,4,5-tetrazin-3-yl group, and the like which may be substituted by the substituent R⁹ described above.

The acyl group represented by the groups R⁴ and R⁵ includes for example a C₂-C₁₀ alkanoyl group such as an acetyl group and a C₂-C₁₀ haloalkanoyl group such as trifluoroacetyl group, the primary or secondary alkyl group which may be substituted includes for example a primary or secondary C₁-C₁₀ alkyl group such as a methyl group, an ethyl group and a propyl group, and the alkylene group which may be substituted includes for example a C₄-C₅ alkylene group such as a tetramethylene group and a pentamethylene group, which may be substituted by a halogen atom such as a fluorine atom, a chlorine atom and a bromine atom, or a C₁-C₃ alkyl group such as a methyl group, an ethyl group, a propyl group or an isopropyl group.

The hydrocarbon group which may be substituted for R⁶ includes for example a C₁-C₆ alkyl group such as a methyl group, an ethyl group and a propyl group, or a phenyl group which may be substituted by a C₁-C₃ alkyl group such as a methyl group, an ethyl group, a propyl group and an isopropyl group, a C₁-C₃ haloalkyl group such as a trifluoromethyl group, a C₁-C₃ alkoxy group such as a methoxy group, an ethoxy group, a propoxy group and an isopropoxy group, a C₁-C₃ haloalkoxy group such as a trifluoromethoxy group and a difluoromethoxy group, a hydroxy group, a nitro group, or a cyano group, or a pyridyl group which may be substituted by a C₁-C₃ alkyl group such as a methyl group, an ethyl group, a propyl group and an isopropyl group, a C₁-C₃ haloalkyl group such as a trifluoromethyl group, a C₁-C₃ alkoxy group such as a methoxy group, an ethoxy group, a propoxy group and an isopropoxy group, a C₁-C₃ haloalkoxy group such as a trifluoromethoxy group and a difluoromethoxy group, a hydroxy group, a nitro group, or a cyano group. The alkoxy group represented by the group R⁶ includes a C₁-C₆ alkoxy group such as a methoxy group, an ethoxy group and a propoxy group, and the mono- or di-alkylamino group includes a mono- or di-(C₁-C₁₀) alkylamino group such as a methylamino group and a dimethylamino group.

The alkyl group represented by R⁷ includes for example a C₁-C₆ alkyl group such as a methyl group, an ethyl group, a propyl group and an isopropyl group.

The present compound can be prepared by the following preparation methods.

Among the present compounds, the compound in which R⁴ and R⁵ represent a hydrogen atom, and R² represents a primary or secondary hydrocarbon group which may be substituted can be prepared by reacting the aminopyrazoline compound represented by the aforementioned formula (II) with the compound represented by the formulae

R²¹-X (III),

R²¹OSO₂CH₃ (IV),

or

(R²¹)₂SO₄ (V),

wherein R²¹ represents a primary or secondary hydrocarbon group which may be substituted, and X represents a chlorine atom, a bromine atom or an iodine atom, ordinarily in an amount of 1.0 - 5.0 equivalents to the aminopyrazoline compound usually in a solvent in the presence of usually 1.0 - 10.0 equivalents of an acid removing agent usually at 0°C - 120°C usually for 1 hour - 100 hours. By way of example, the hydrocarbon group represented by R²¹ includes the primary or secondary ones among those described above for R².

As the solvent, there are mentioned for example aromatic hydrocarbons such as benzene, toluene and xylene, ethers such as diethyl ether, diisopropyl ether, dioxane, tetrahydrofuran and ethylene glycol dimethyl ether, ketones such as acetone, methyl ethyl ketone, methyl isobutyl ketone, isophorone and cyclohexanone, alcohols such as methanol, ethanol, isopropyl alcohol, tert-butyl alcohol and diethylene glycol, nitriles such as acetonitrile, acidamides such as N,N-dimethylacetamide, sulfur compounds such as dimethylsulfoxide and sulfolane, or the mixtures thereof. The acid removing agent includes for example organic bases such as pyridine and triethylamine, or inorganic bases such as sodium carbonate, potassium carbonate, sodium hydroxide, lithium hydroxide and sodium hydride.

After the completion of the reaction, the reaction mixture can be subjected to the ordinary post-treatments such as extraction with organic solvent and concentration, and, if necessary, further purified by chromatography, recrystallization, or the like to isolate the desired present compound.

Also, among the present compounds, the compound in which R⁴ and R⁵ represent a hydrogen atom can be prepared by reacting the compound represented by the formula (VI): 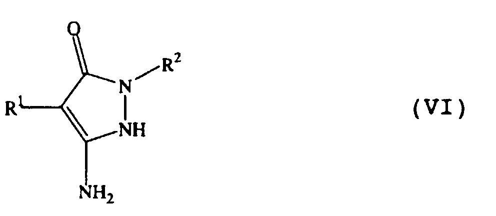 wherein R¹ and R² have the same meanings as defined above, with the compound represented by the formula (VII): 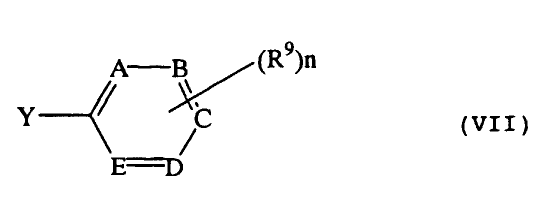 wherein R⁹ represents a C₁-C₆ alkyl group, a C₁-C₆ alkoxy group, a C₁-C₆ alkylthio group, a C₁-C₆ haloalkyl group, a C₁-C₆ haloalkoxy group, a C₁-C₆ haloalkylthio group, or a halogen atom, A, B, C, D and E, which may be the same or different, represent a CH group or a nitrogen atom, respectively. n represents an integer of 0 - 4, when either one of A - E represents a nitrogen atom, n represents an integer of 0 - 3, when either two of A - E represent a nitrogen atom, n represents an integer of 0 - 2, when either three of A - E represent a nitrogen atom, and n represents an integer of 0 - 1, when either four of A - E represent a nitrogen atom, Y represents a halogen atom such as a chlorine atom, a bromine atom, an iodine atom or fluorine atom, or a methanesulfonyl group, usually in a solvent in the presence of usually 1.0 - 10.0 equivalents of an acid removing agent usually at 0°C - 120°C usually for 1 hour - 100 hours. According to this method, not only the compounds in which R² represents a primary or secondary hydrocarbon group which may be substituted, but also the compounds in which R² represents a tertiary hydrocarbon group which may be substituted can be prepared.

As the solvent, there are mentioned for example aromatic hydrocarbons such as benzene, toluene and xylene, ethers such as diethyl ether, diisopropyl ether, dioxane, tetrahydrofuran and ethylene glycol dimethyl ether, ketones such as acetone, methyl ethyl ketone, methyl isobutyl ketone, isophorone and cyclohexanone, alcohols such as methanol, ethanol, isopropyl alcohol, tert-butyl alcohol and diethylene glycol, nitriles such as acetonitrile, acid amides such as N,N-dimethylacetamide, sulfur compounds such as dimethylsulfoxide and sulfolane, or the mixtures thereof. The acid removing agent includes for example organic bases such as pyridine and triethylamine, or inorganic bases such as sodium carbonate, potassium carbonate, sodium hydroxide, lithium hydroxide and sodium hydride.

After the completion of the reaction, the ordinary post-treatments such as extraction with organic solvent and concentration are conducted, and, if necessary, further purified by chromatography, recrystallization, or the like to isolate the present compound as the preferred product.

Also, among the present compounds, the compound in which at least one of R⁴ or R⁵ does not represent a hydrogen atom can be prepared by reacting the present compound in which R⁴ and R⁵ both represent a hydrogen atom with the compound

R⁴¹-X (VIII),

(R⁴¹)₂SO₄ (IX),

R¹⁰COCl (X),

(R¹⁰CO)₂O (XI),

or

X-R⁴²-R⁵²-X (XII)

wherein R⁴¹ represents a primary or secondary alkyl group which may be substituted, R¹⁰ represents an acyl moiety from which the carbonyl group has been removed, R⁴²-R⁵² represents an alkylene group which may be substituted, and X has the same meanings as defined above, usually in a solvent in the presence of usually 1.0 - 5.0 equivalents of a base and usually 0.1 - 1.0 equivalent of a phase-transfer catalyst usually at 0°C - 100°C usually for 1 hour - 100 hours.

As the solvent used, there are mentioned for example aromatic hydrocarbons such as benzene, toluene and xylene, halogenated hydrocarbons such as dichloromethane, chloroform, carbon tetrachloride, dichloroethane and chlorobenzene, ethers such as diethyl ether, diisopropyl ether, dioxane, tetrahydrofuran and ethylene glycol dimethyl ether, ketones such as acetone, methyl ethyl ketone, methyl isobutyl ketone, isophorone and cyclohexanone, alcohols such as methanol, ethanol, isopropyl alcohol, tert-butyl alcohol and diethylene glycol, nitriles such as acetonitrile, acid amides such as N,N-dimethylacetamide, water or the like, or the mixtures thereof. The base used includes for example inorganic bases such as sodium carbonate, potassium carbonate, sodium hydroxide, lithium hydroxide and sodium hydride.

As the phase-transfer catalyst, there are mentioned for example quaternary ammonium salts such as tetra-n-butylammonium bromide, tetra-n-butylammonium chloride and benzyltriethylammonium chloride.

After the completion of the reaction, the ordinary post-treatments such as extraction with organic solvent and concentration are conducted, and, if necessary, further purified by chromatography, recrystallization, or the like to isolate the desired present compound.

Next, the specific examples of the present compounds are illustrated below. In the following chemical formulae, (R⁸)m represents the groups listed in Tables 1 or 2 hereunder.

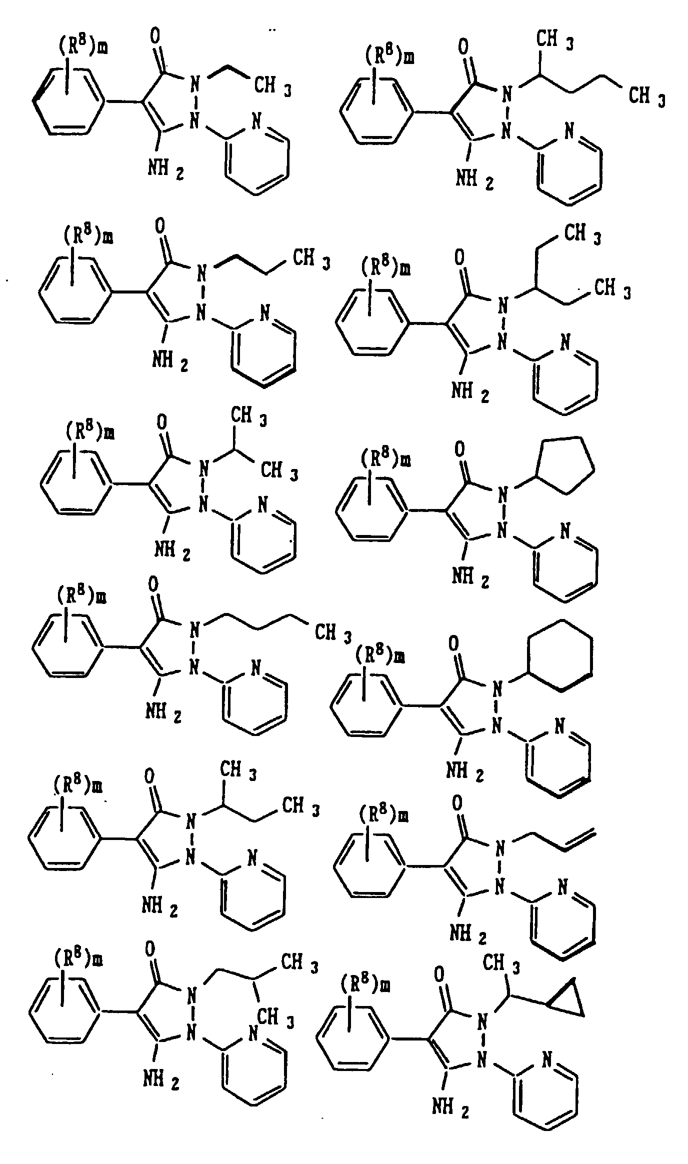

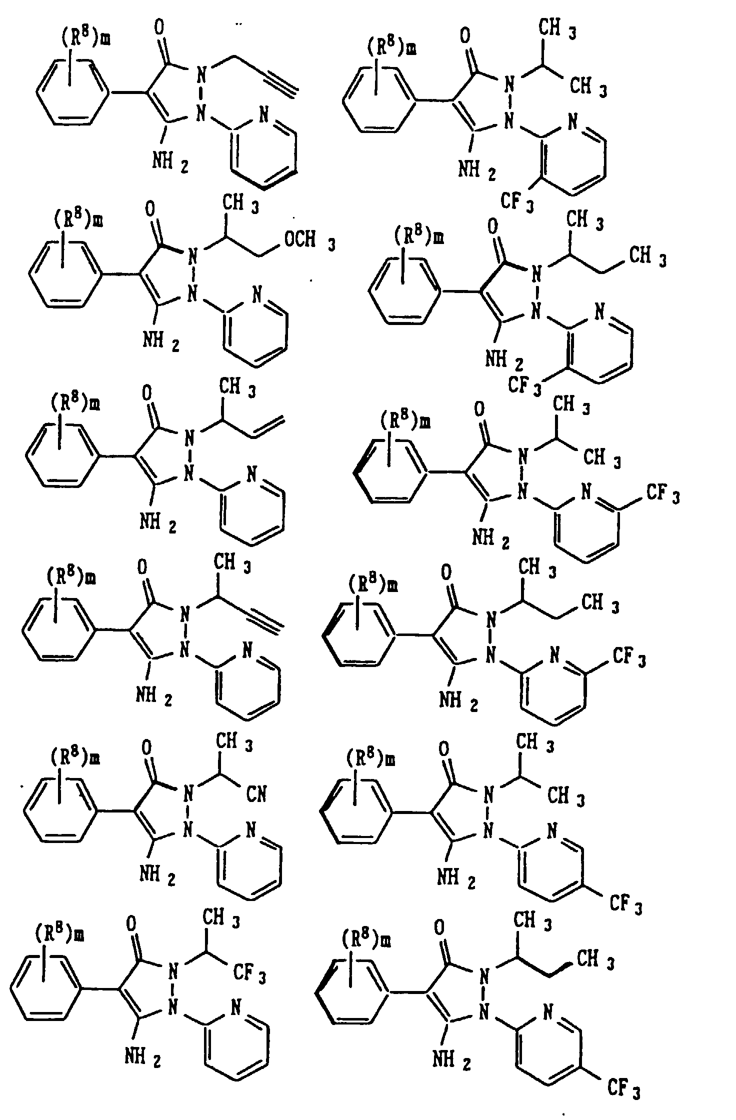

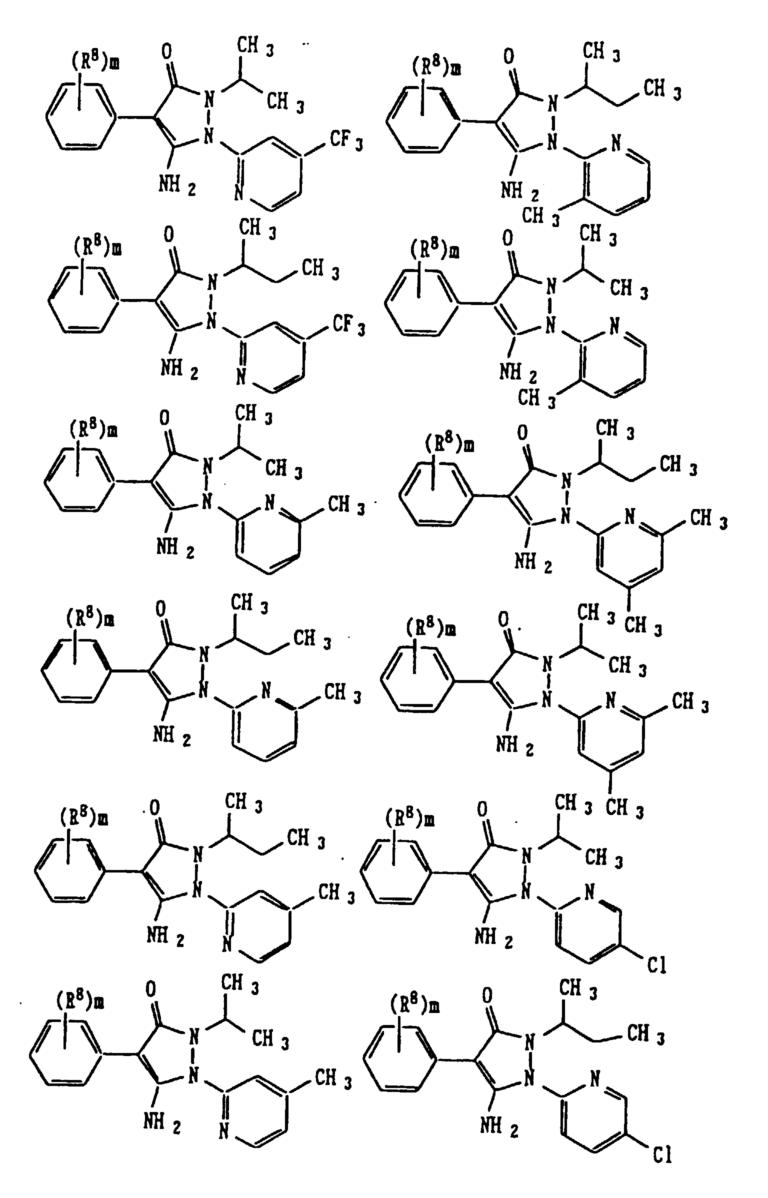

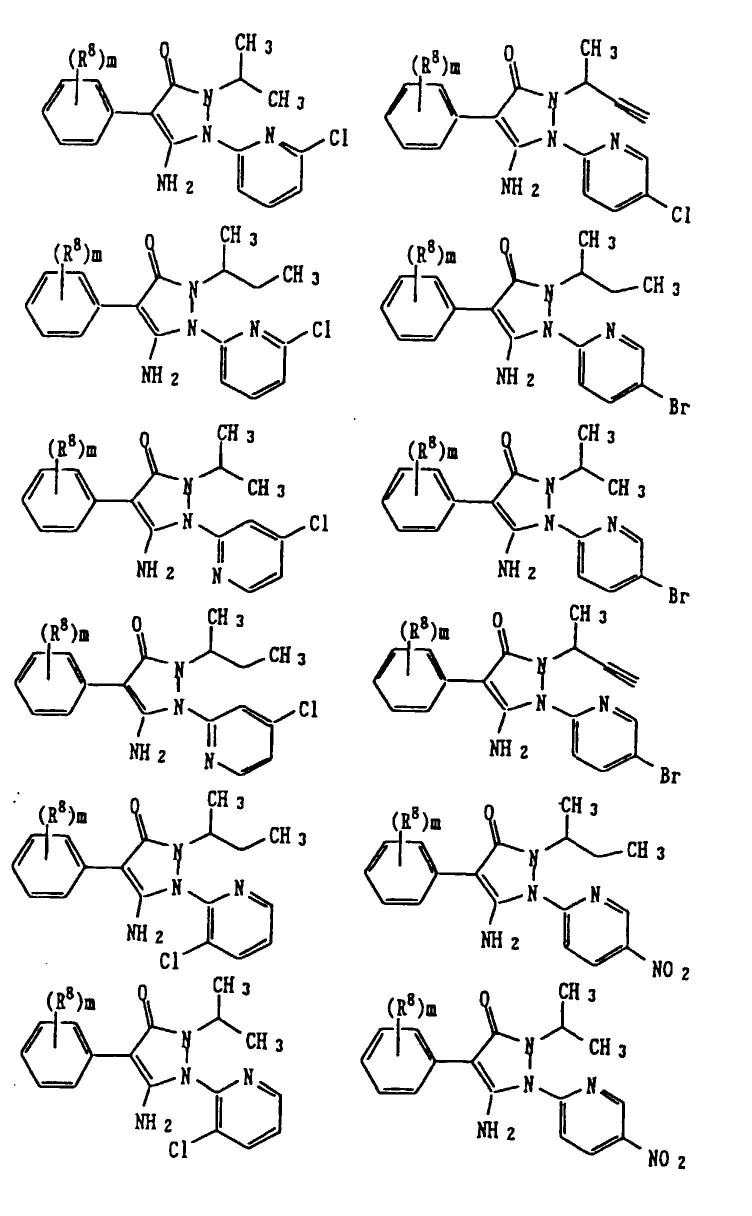

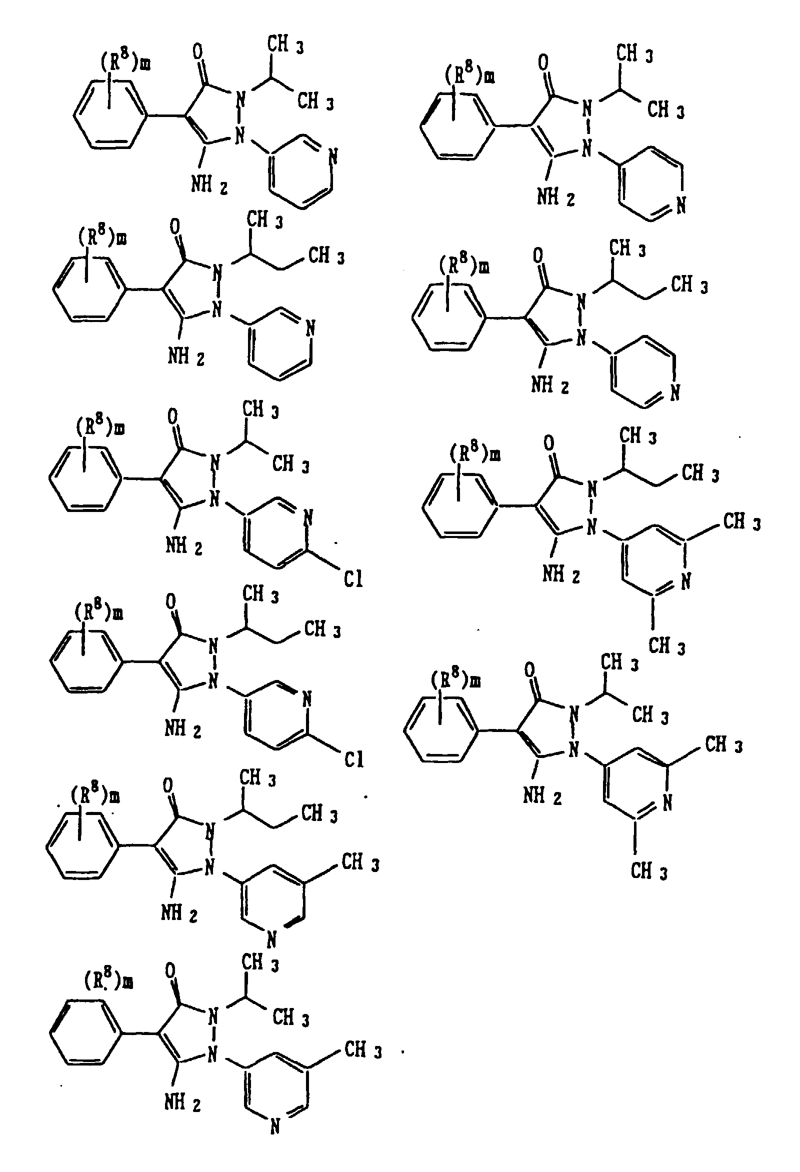

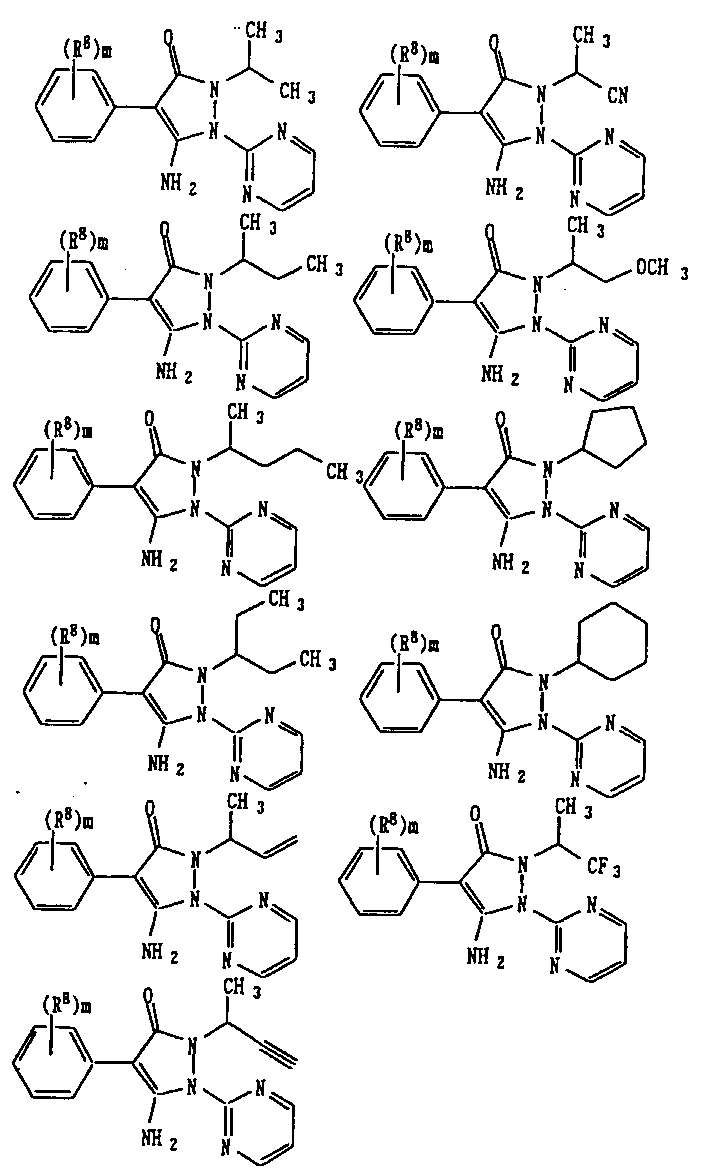

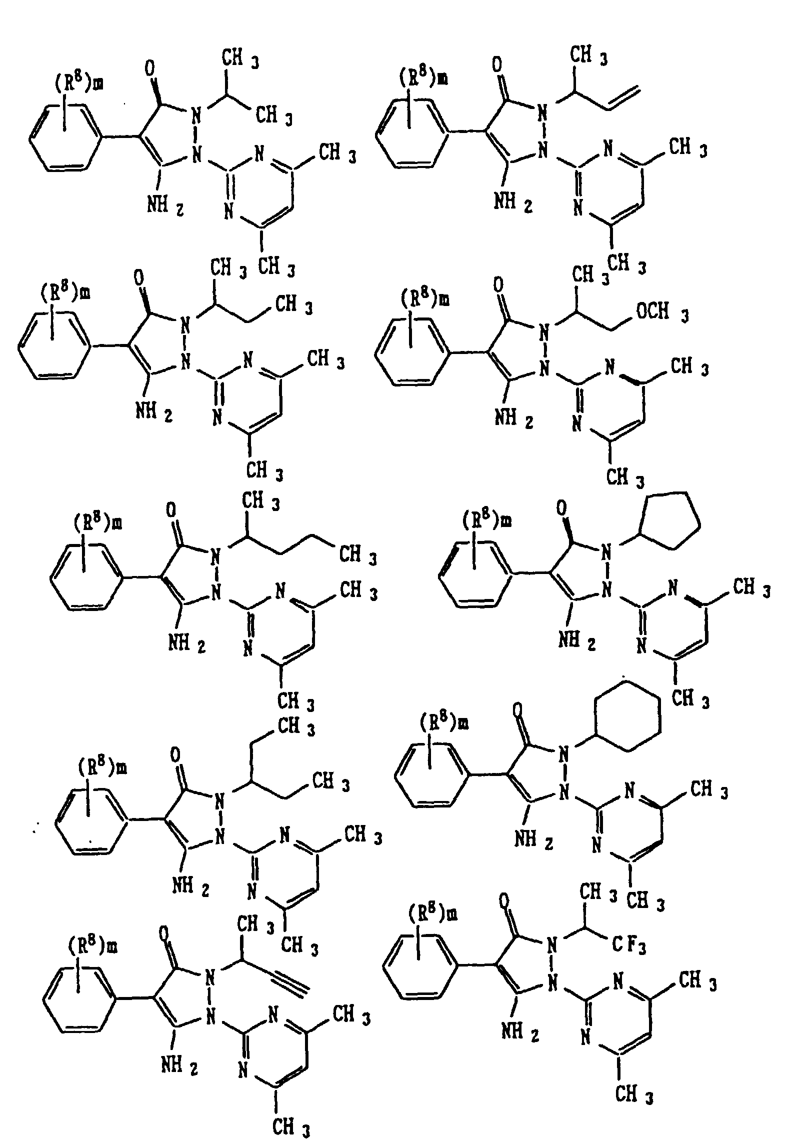

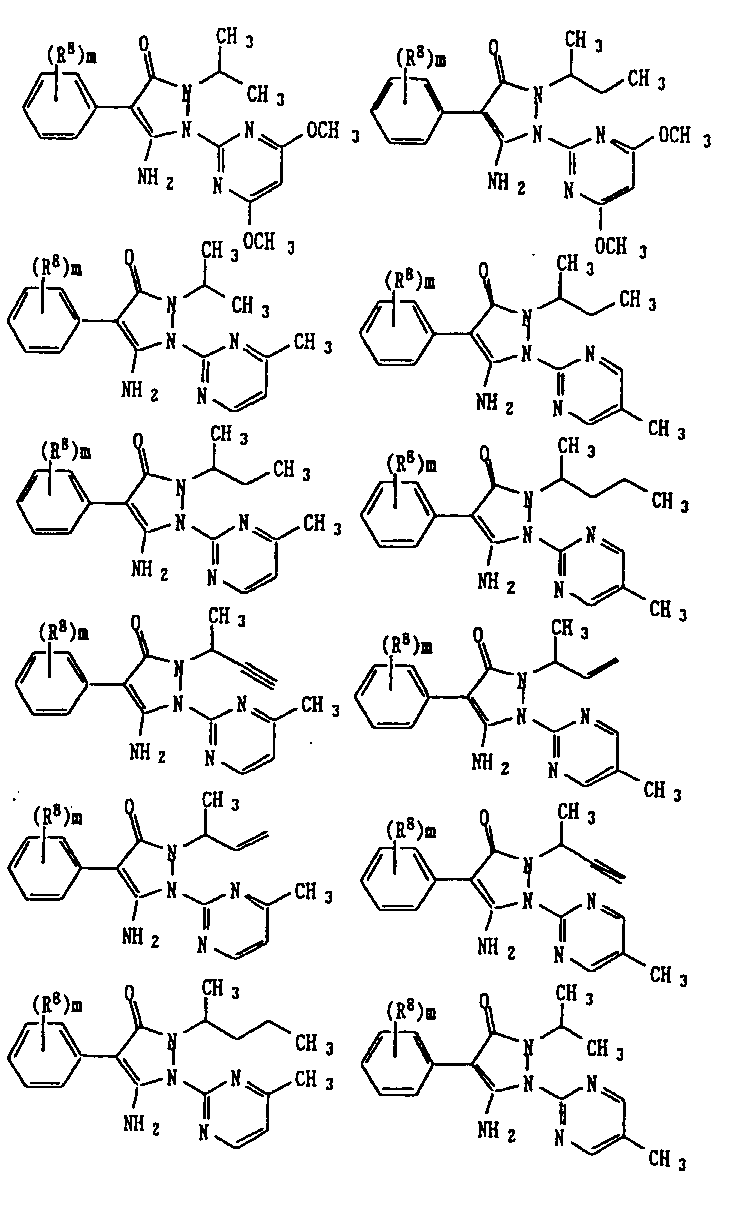

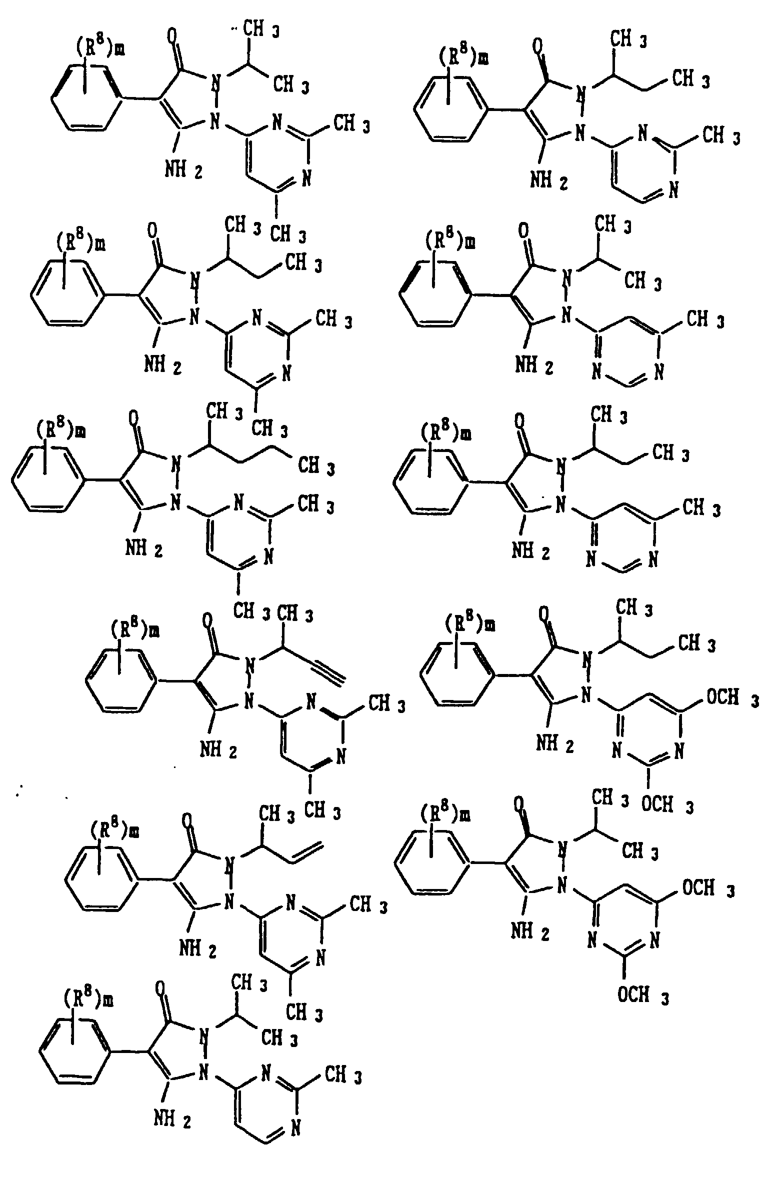

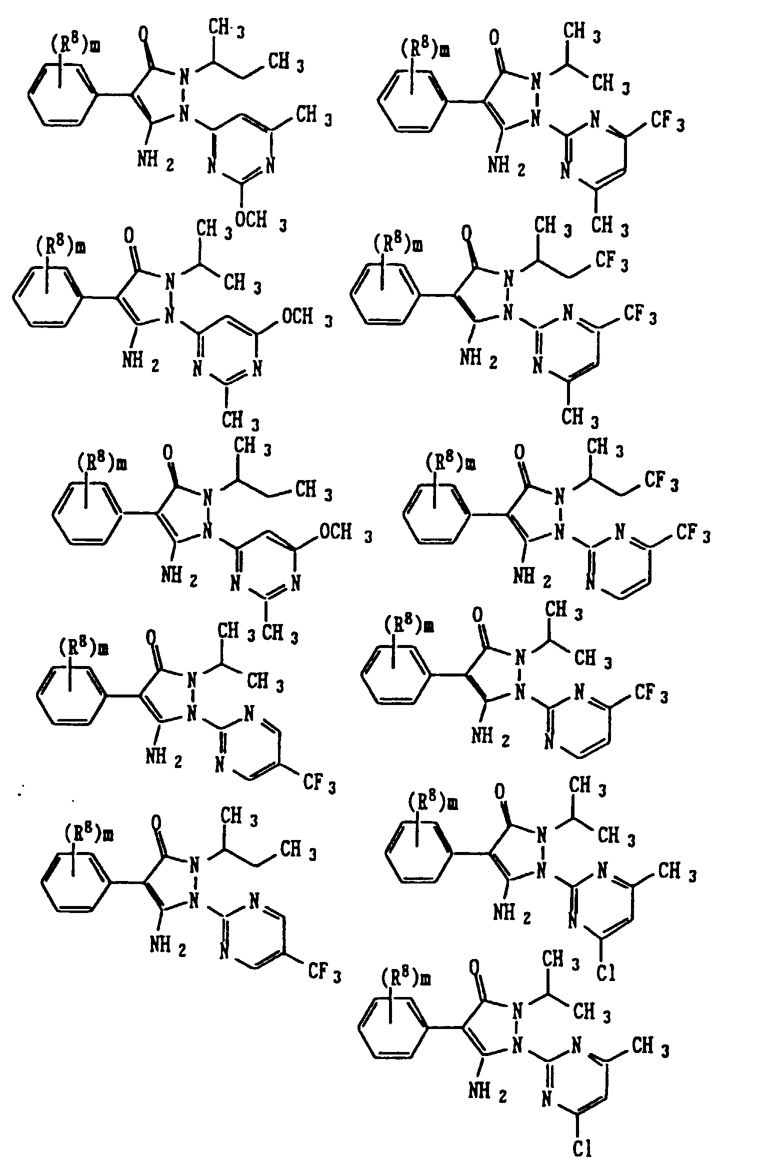

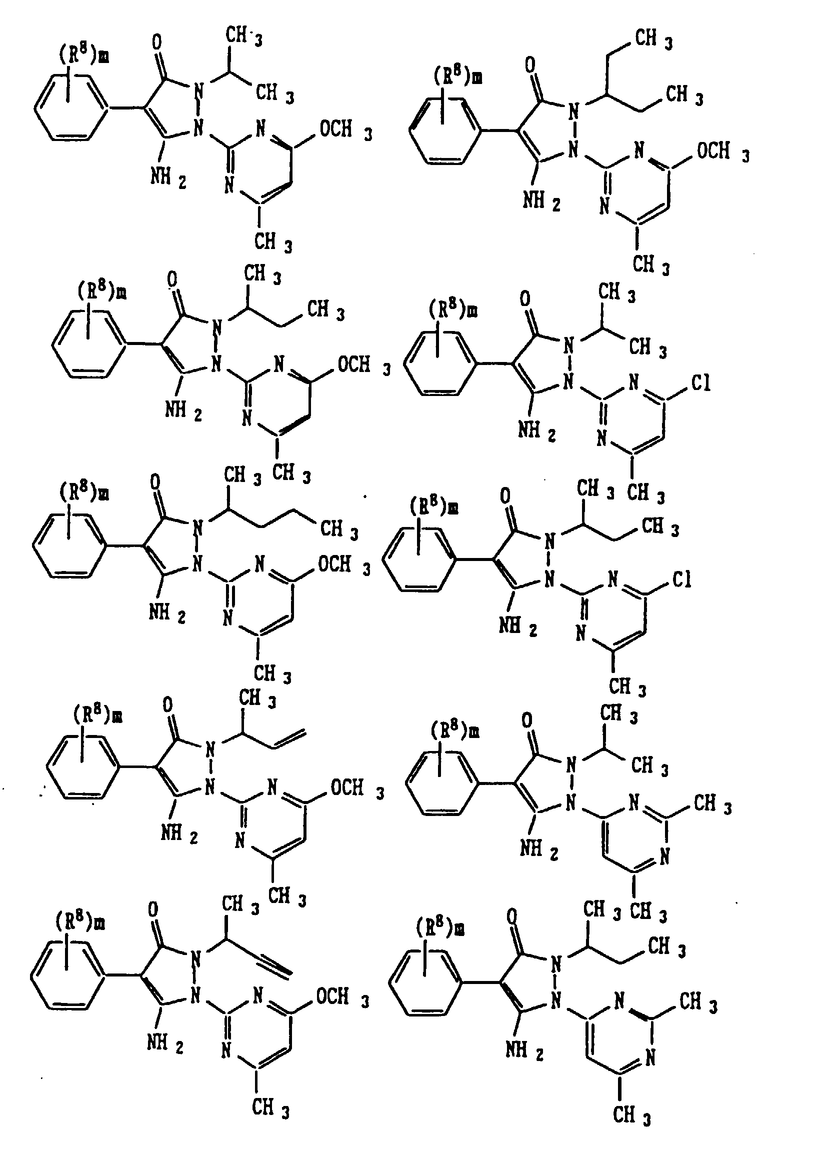

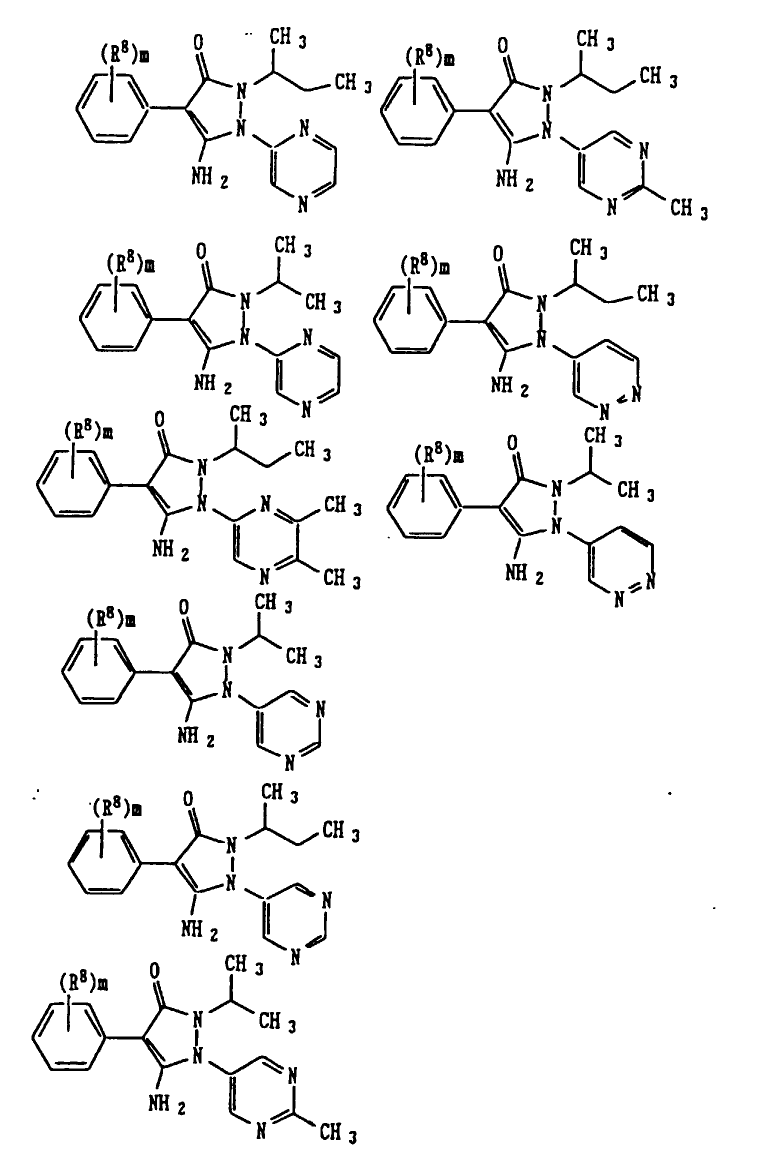

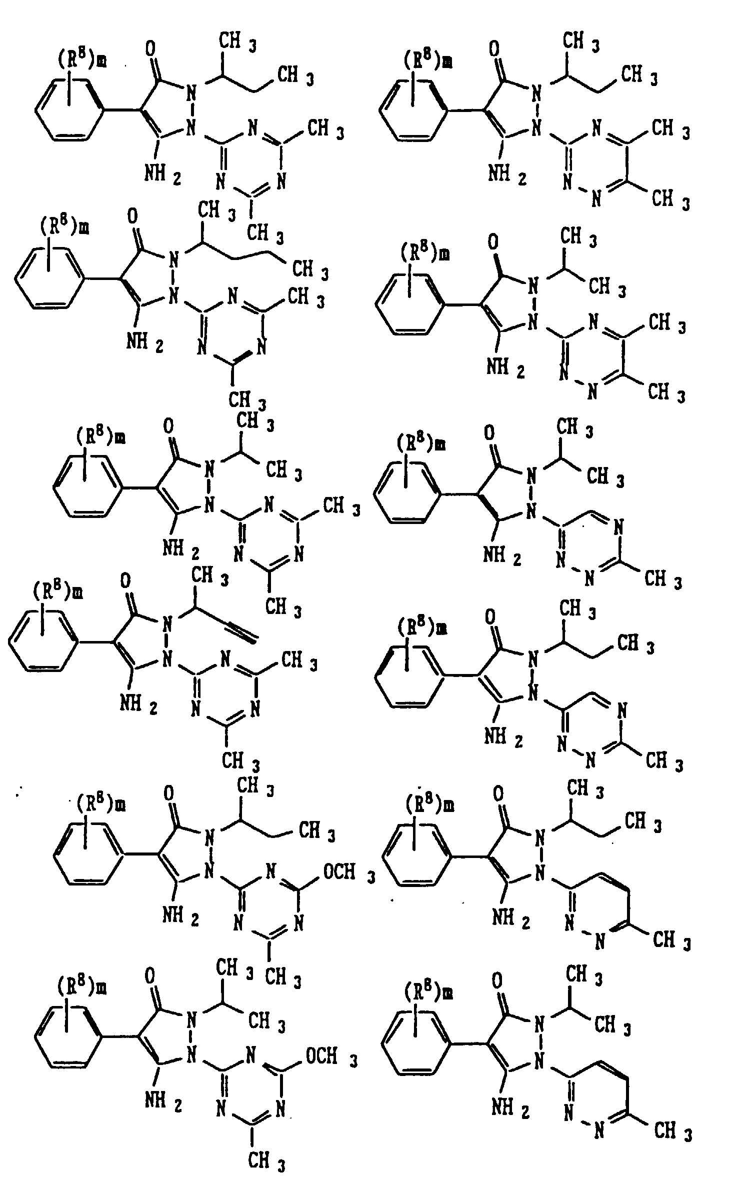

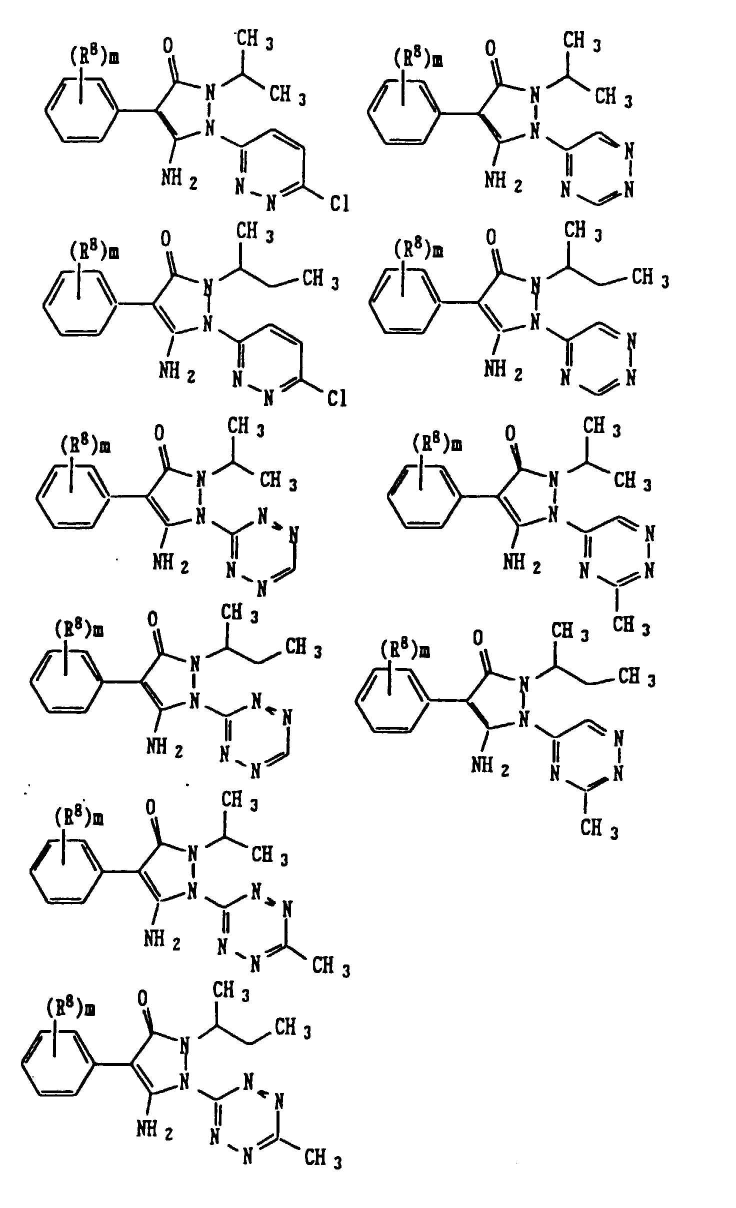

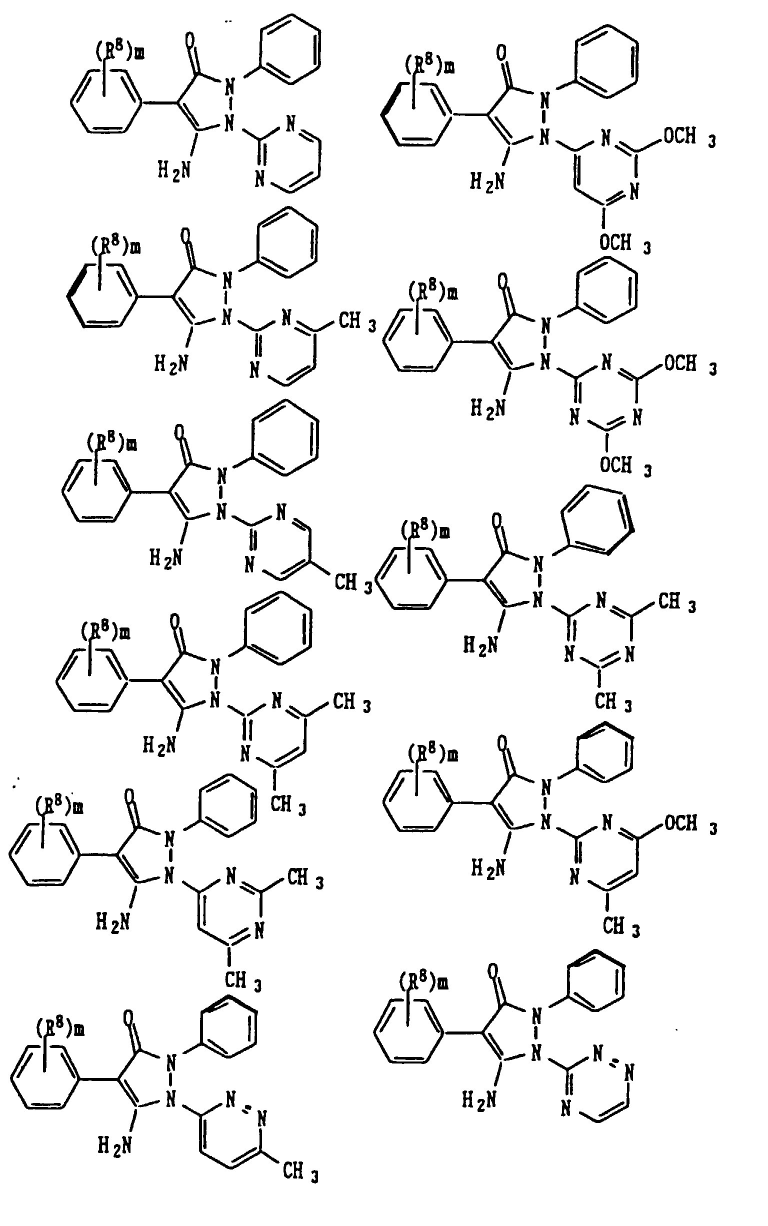

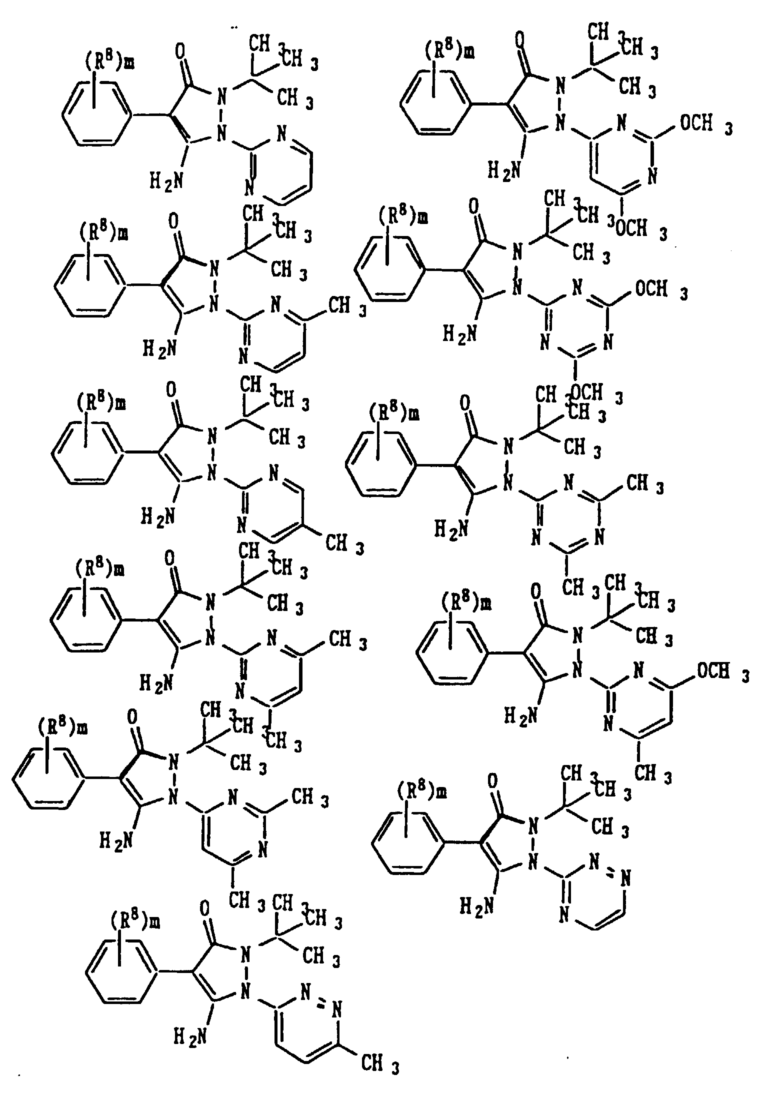

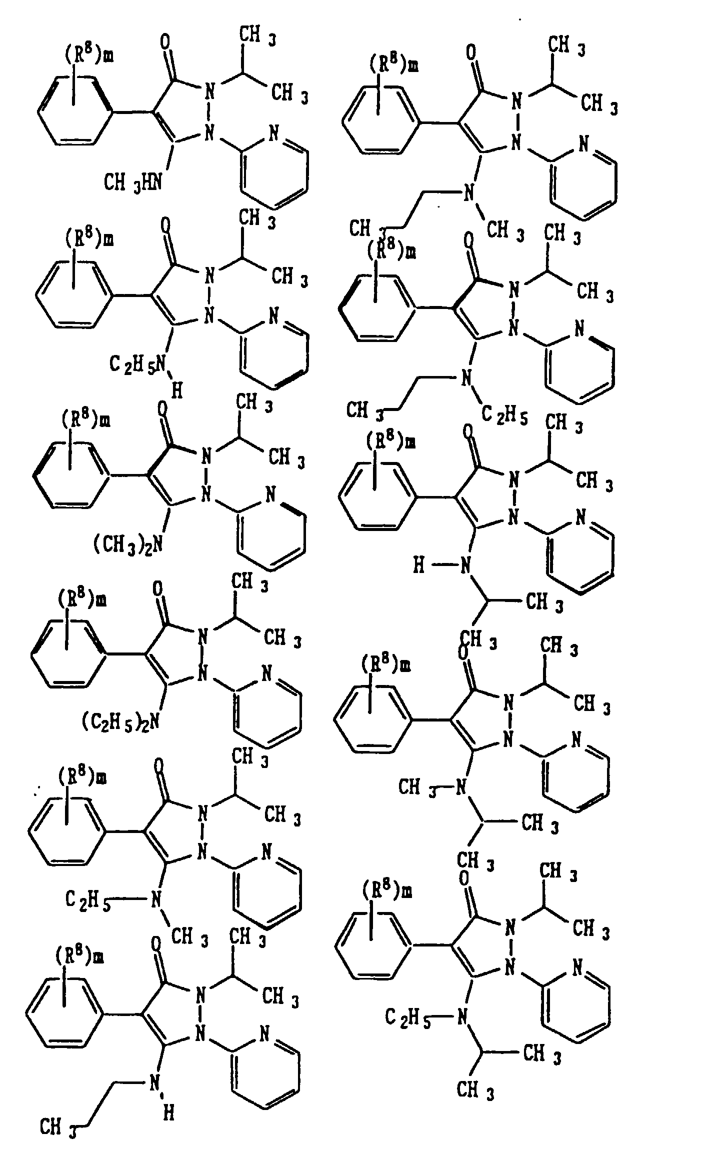

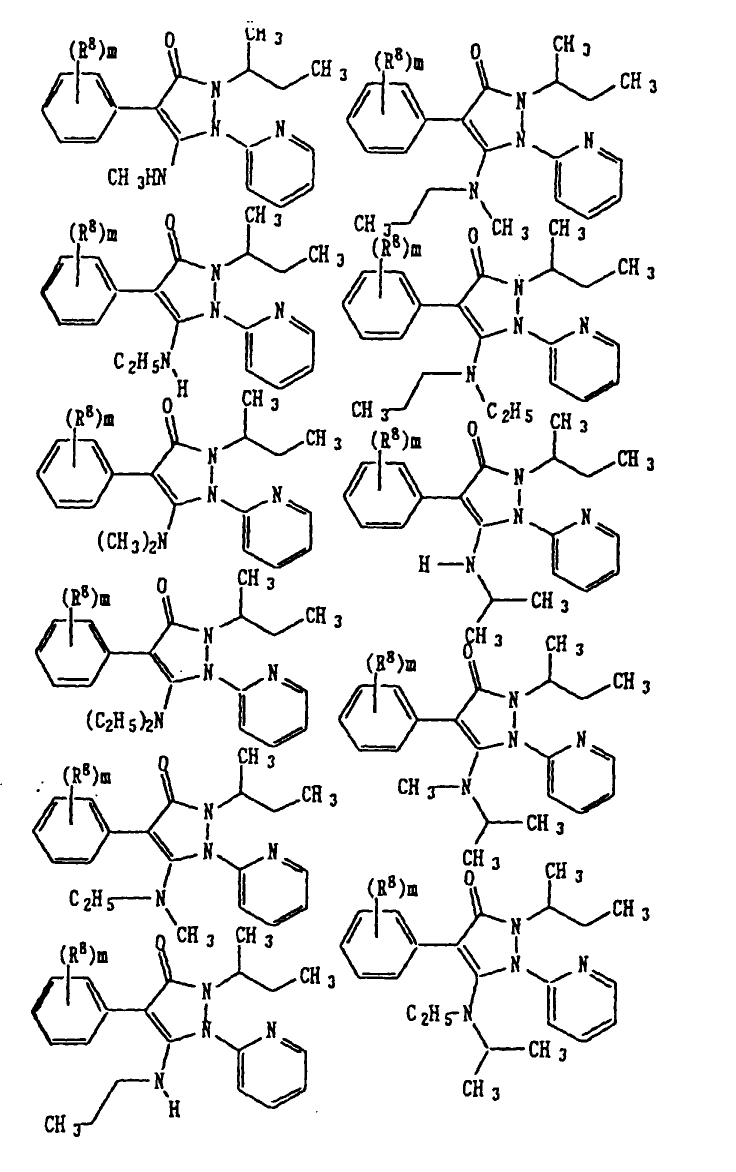

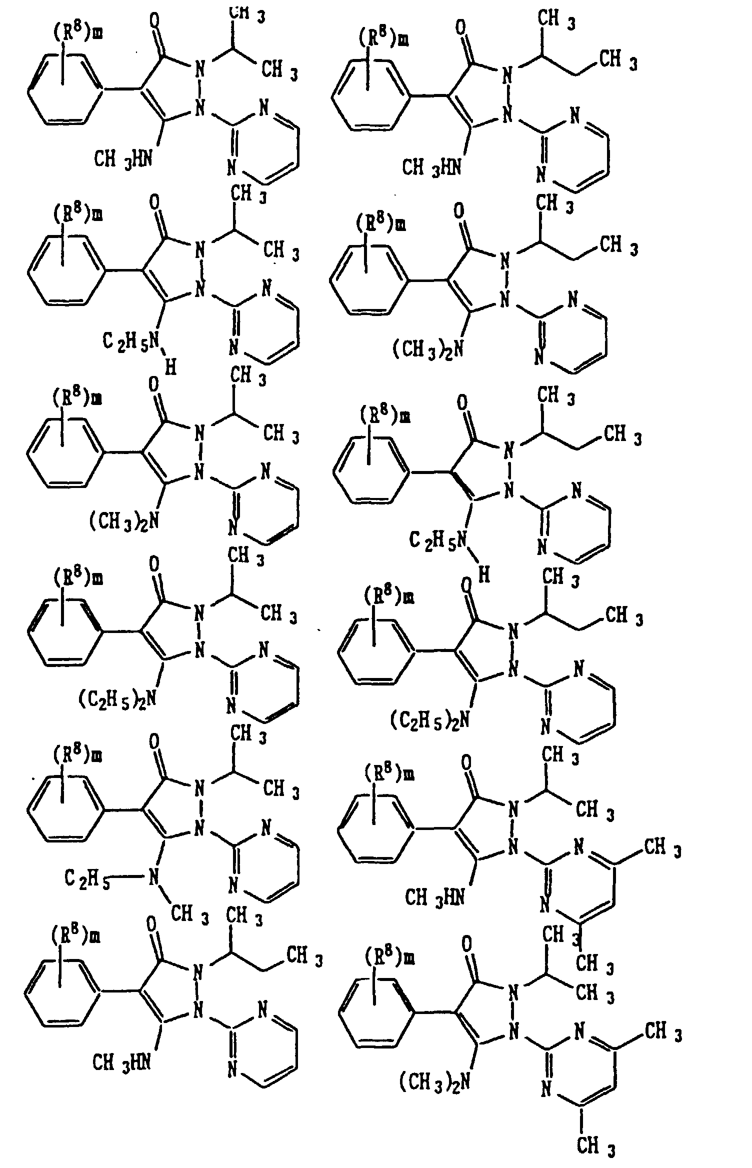

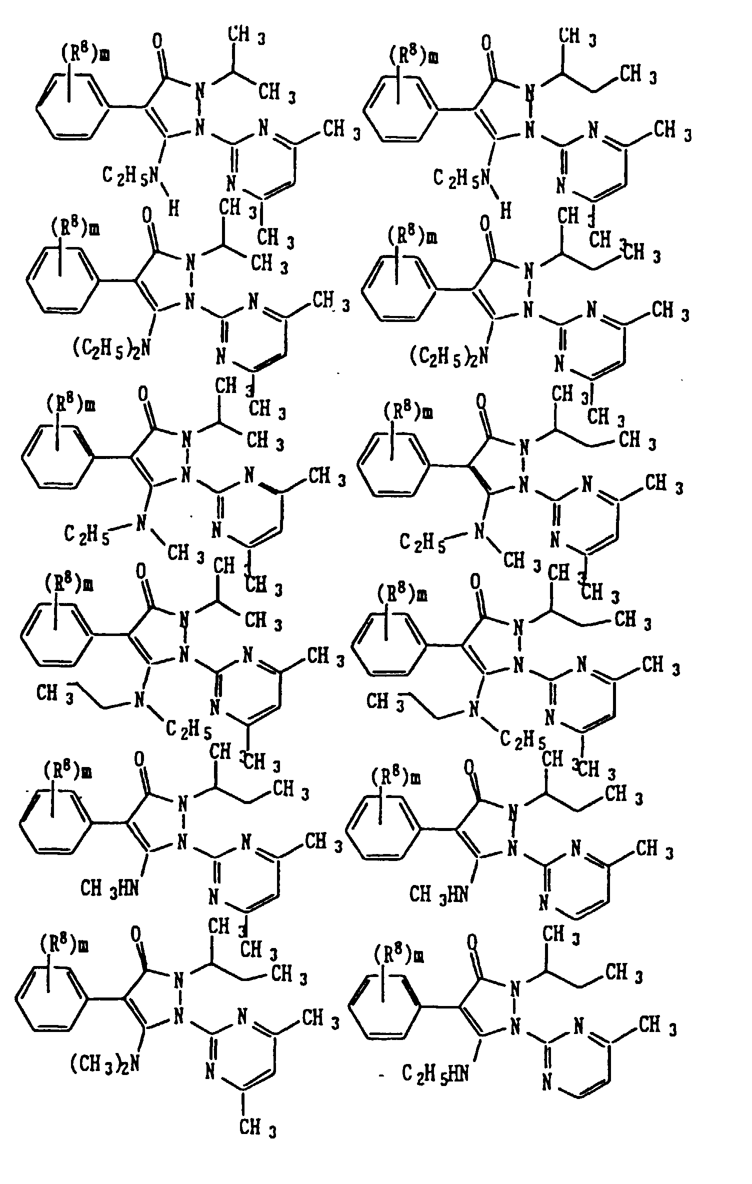

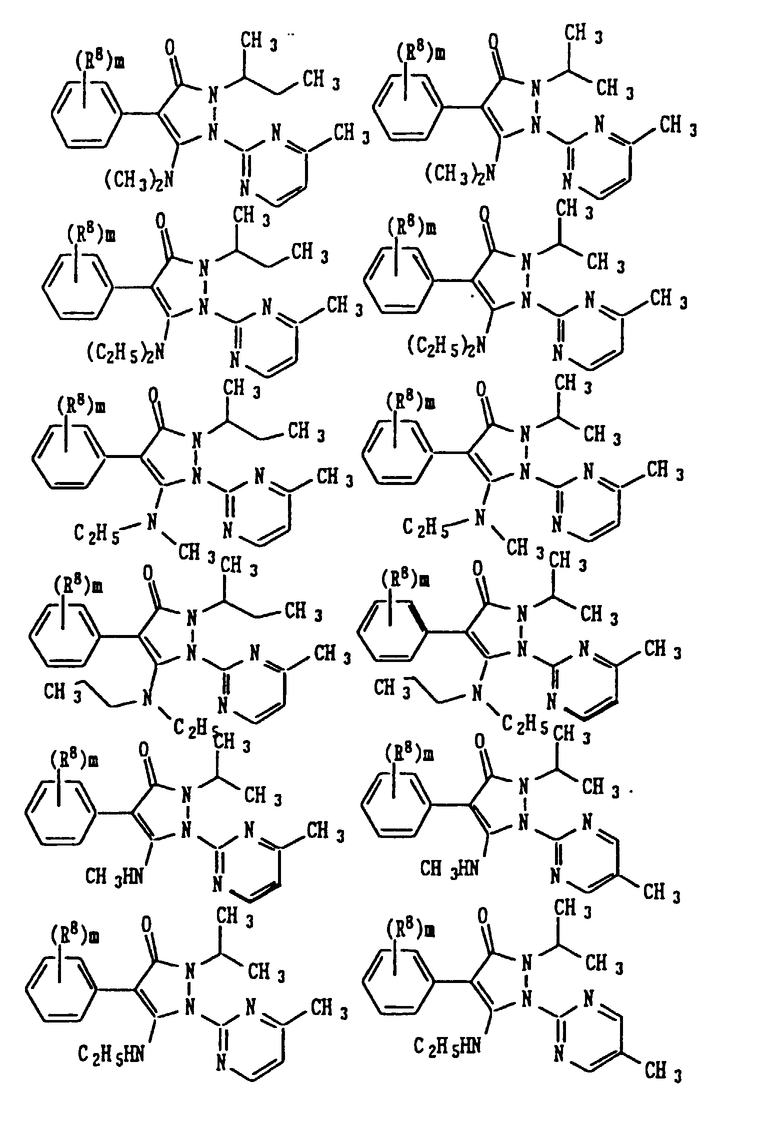

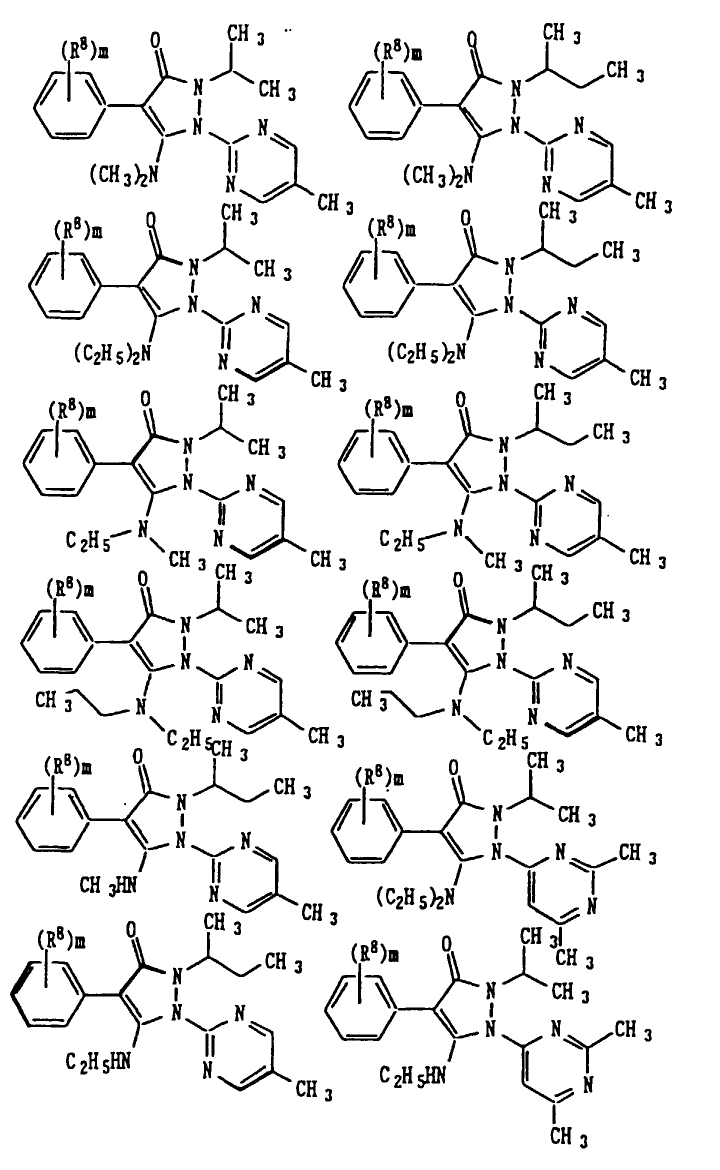

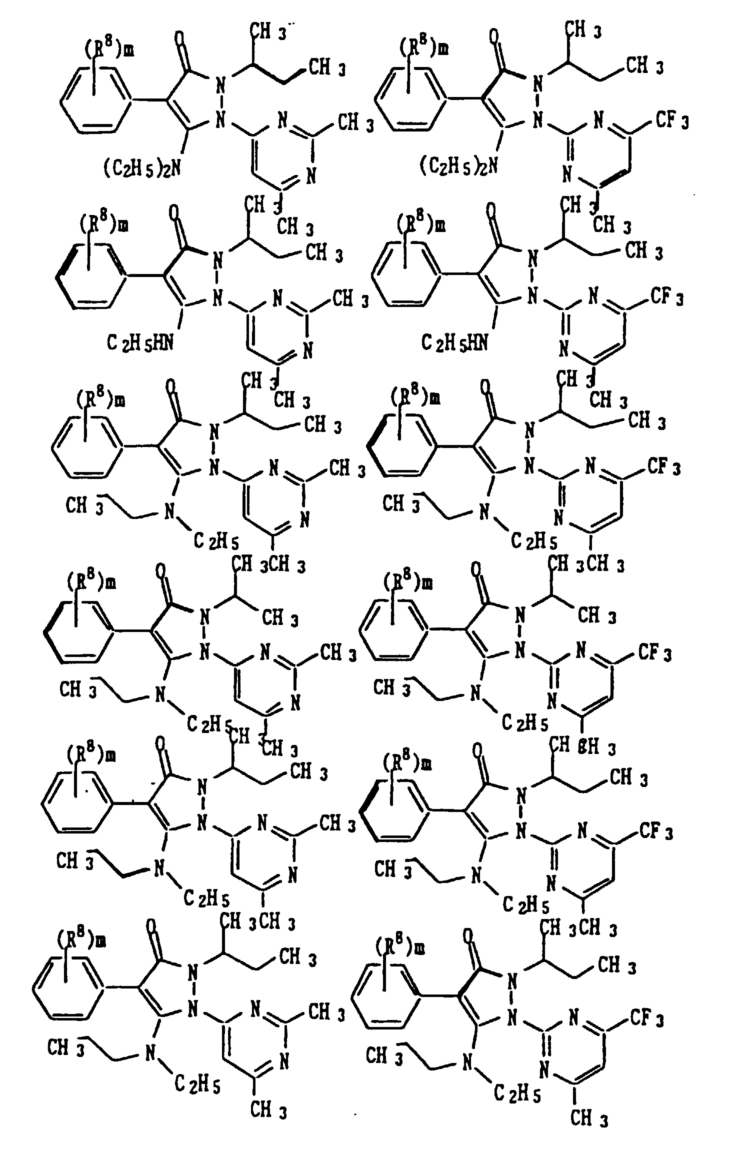

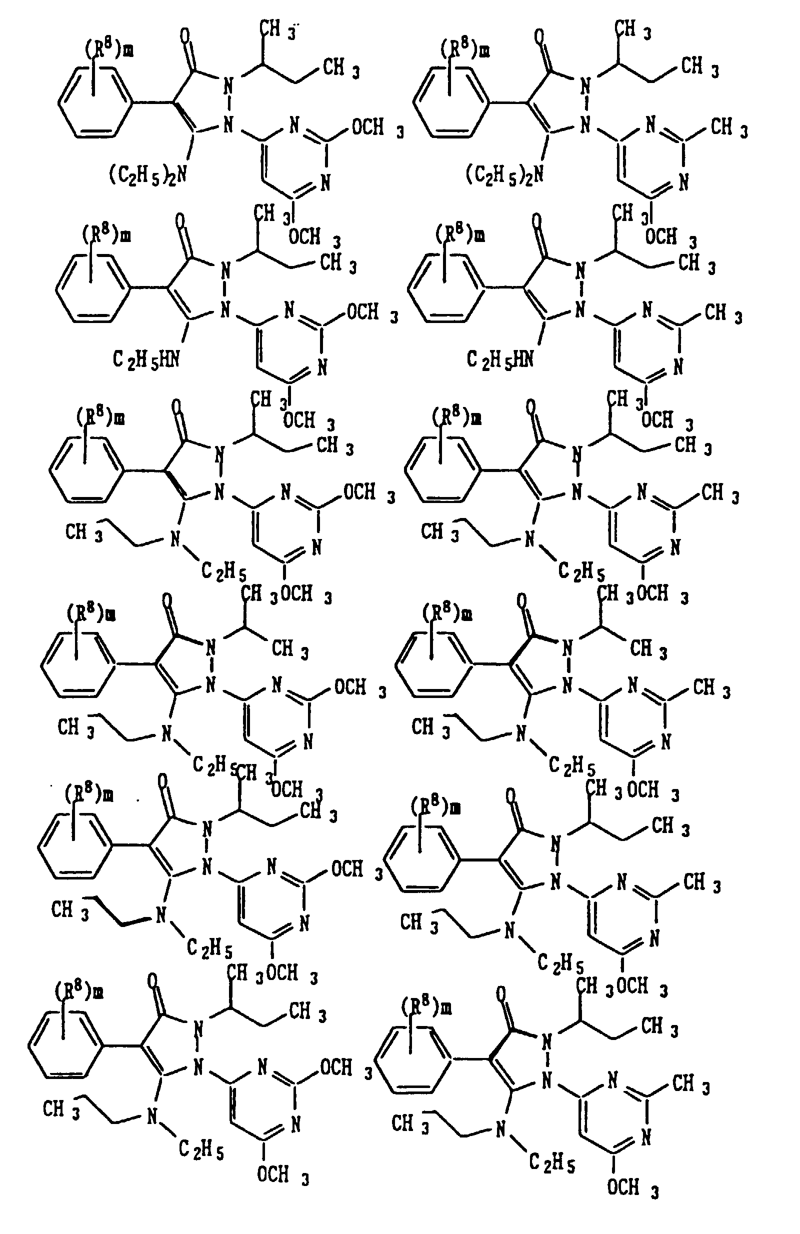

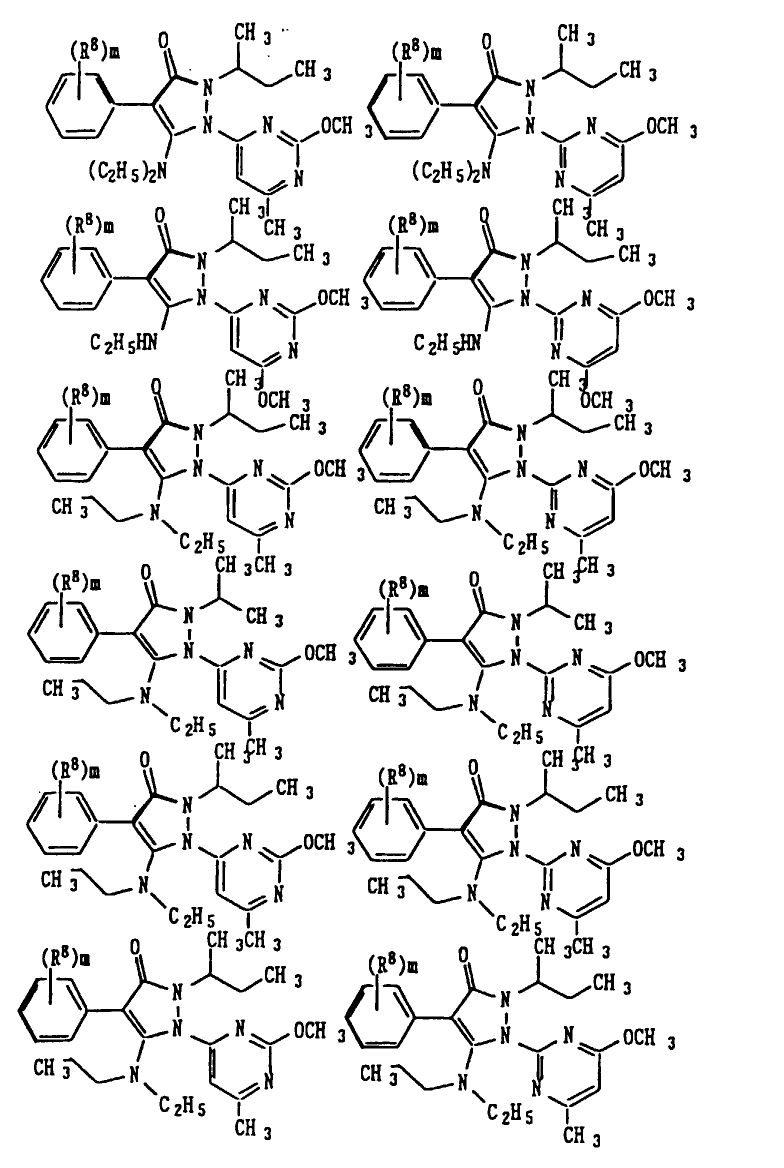

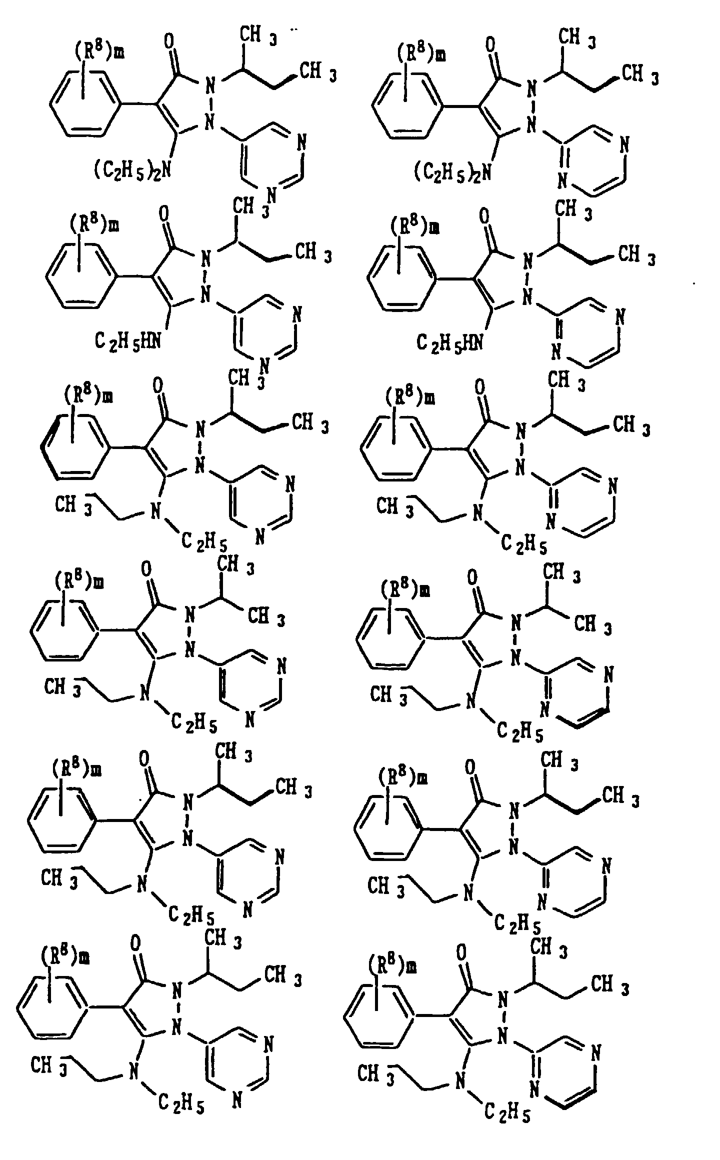

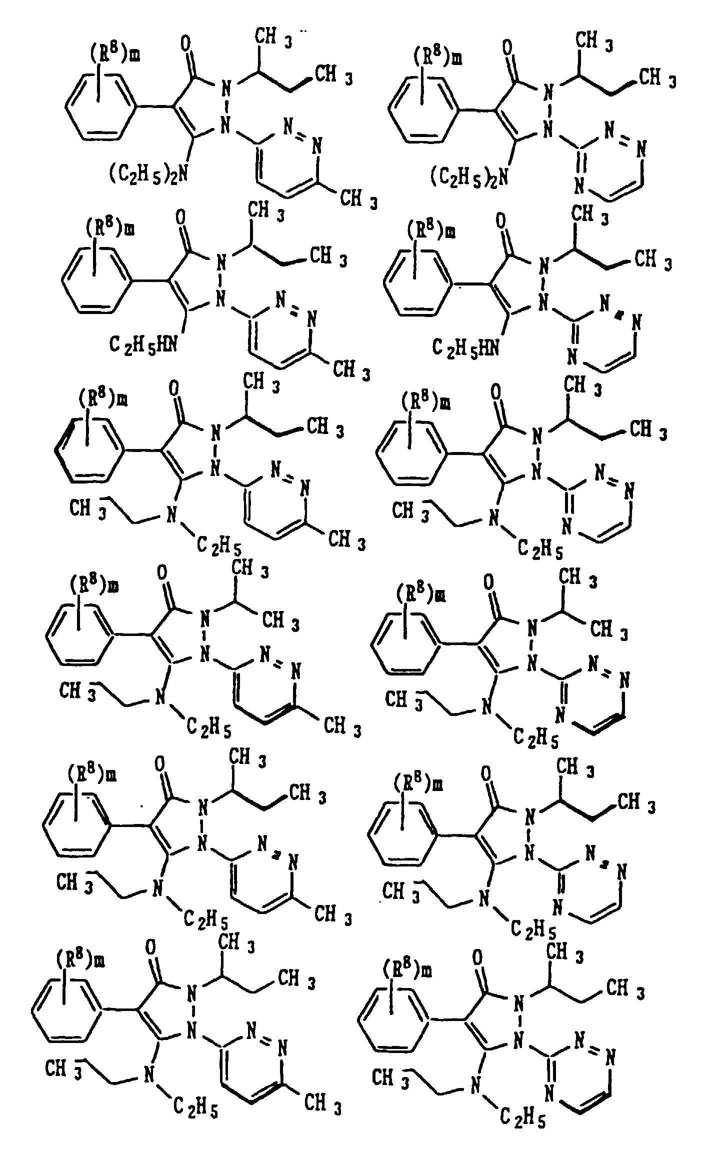

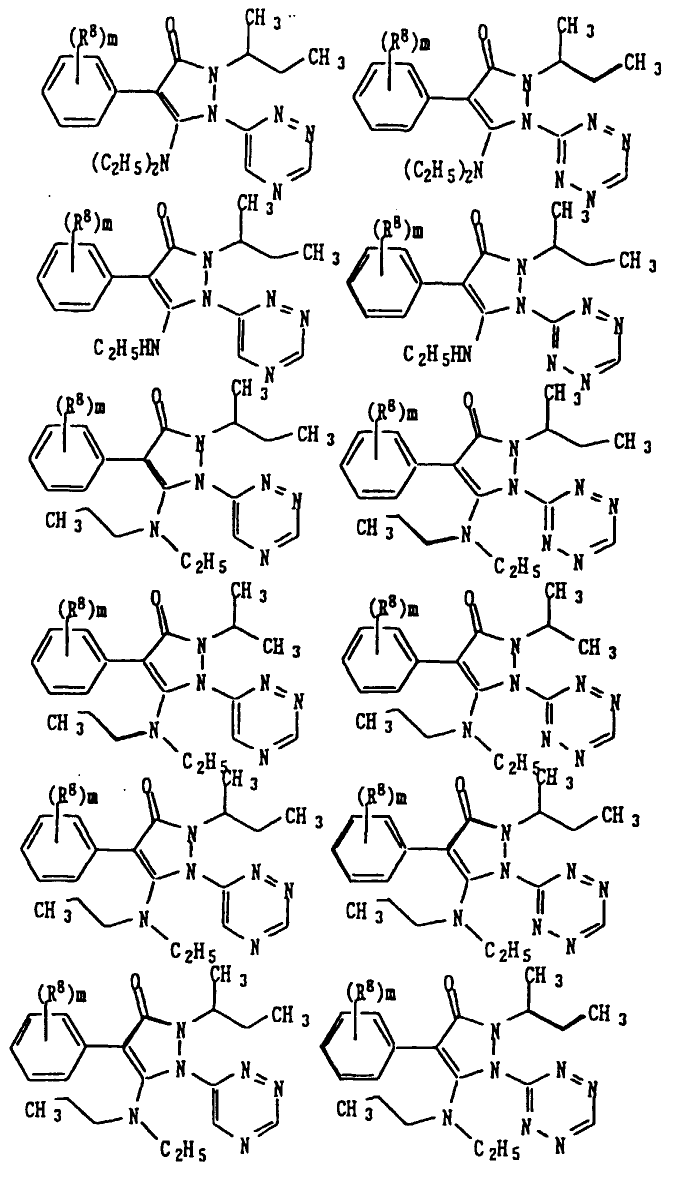

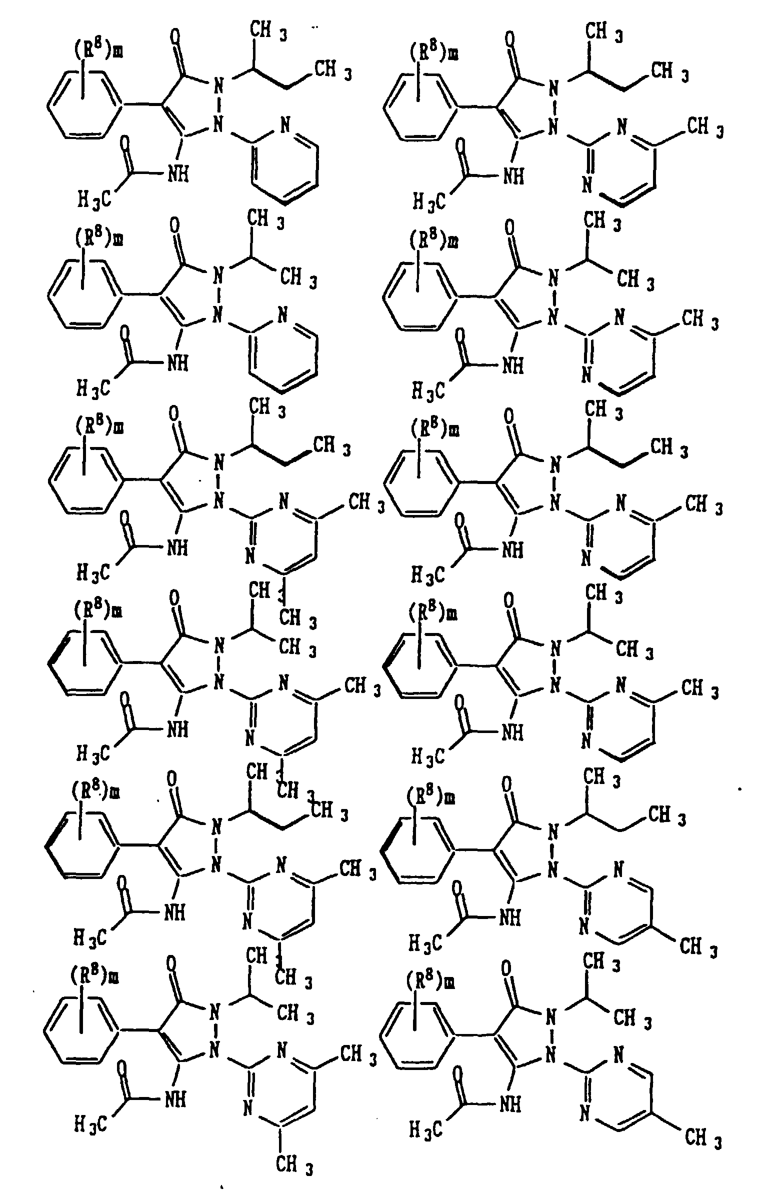

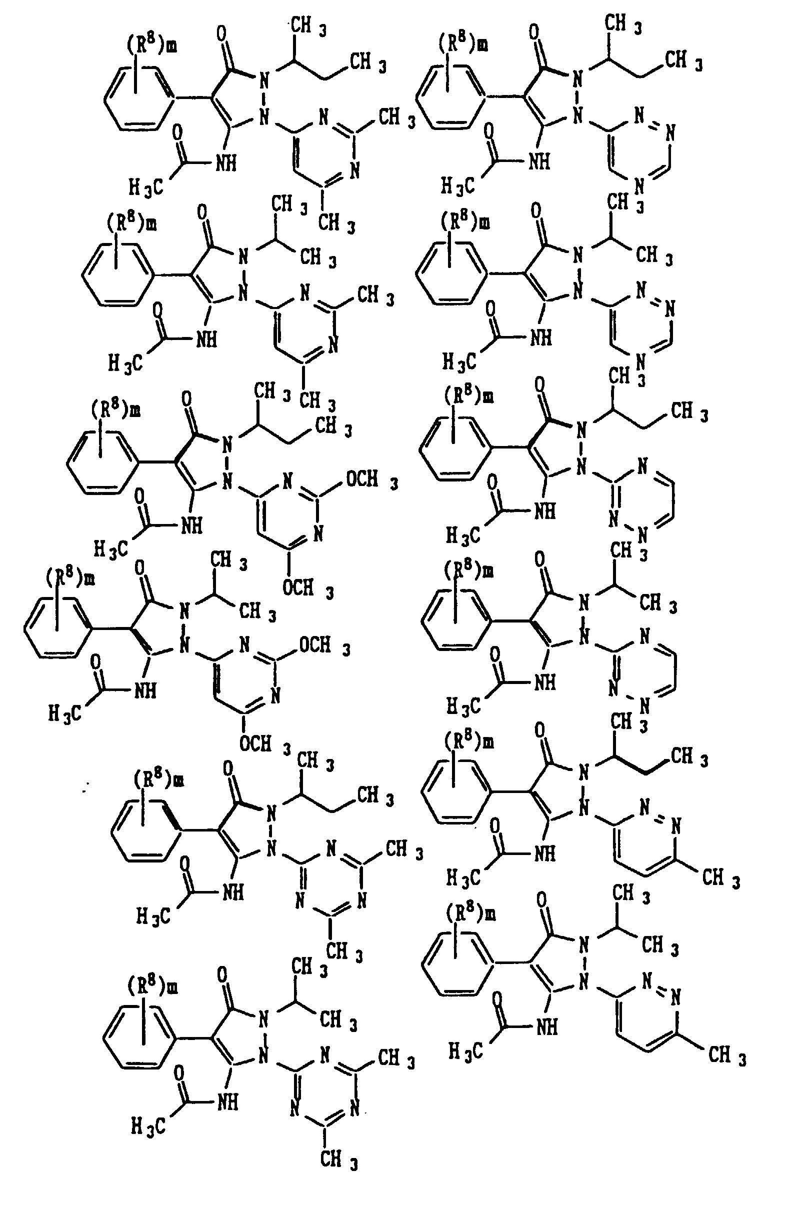

The aminopyrazoline compound represented by the formula (II) can be prepared for example by reacting the phenylcyanoacetic acid ester compound represented by the formula (XIII):

R¹-CH(C≡N)CO₂R (XIII)

wherein R¹ has the same meanings as defined above, and R represents a C₁-C₃ alkyl group such as a methyl group, an ethyl group, a propyl group and an isopropyl group, or a phenyl group, with the aromatic heterocyclic hydrazine compound such as for example the hydrazine compound represented by the formula (XIV): 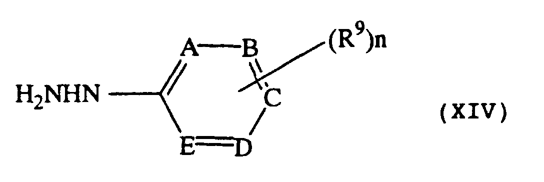 wherein A, B, C, D, E, R⁹, and n have the same meanings as defined above, usually at 20°C - 200°C usually for 1 hour - 50 hours.

While a solvent is not necessarily essential for the reaction, the solvent which can be used includes alcohols such as methanol and ethanol, and aromatic hydrocarbons such as benzene, toluene and xylene in the reaction in solvent.

Also, while a catalyst is not necessarily essential for the reaction, the catalyst which can be used includes acetic acid, p-toluenesulfonic acid, and the like, when the reaction is effected using catalyst.

After the completion of the reaction, the crystallized product is subjected to the ordinary post-treatments such as the washing with an organic solvent, e.g., hexane, extraction with an organic solvent and concentration, and if necessary, further purified by chromatography or recrystallization to isolate the desired compound.

As the phenylcyanoacetic acid ester represented by the formula (XIII), there can be used the commercially available compounds or the ones prepared according to the methods described for example in Chem. Lett., p 193 (1983), J. Org. Chem., 58, 7606 (1993), or Japanese Patent Laid-Open Publication No. 1/160968.

As the hydrazine compound represented by the formula (XIV), there are used the commercially available compounds or the ones prepared for example according to the following schemes (i) or (ii): 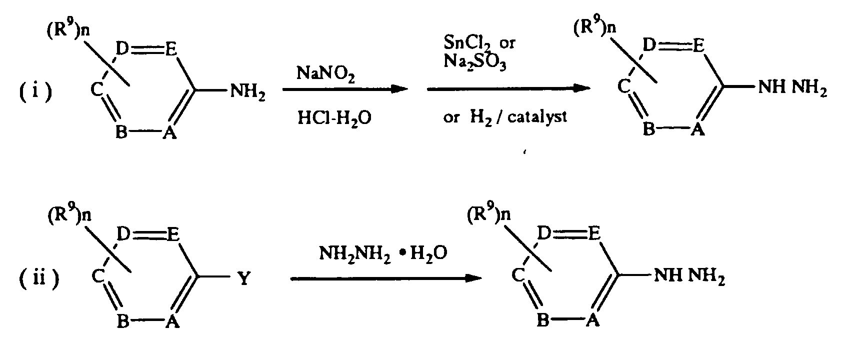 wherein A, B, C, D and E, which may be the same or different, represent a nitrogen atom or a group CH, provided that all of A, B, C, D and E will not represent the group CH at the same time, Y, R⁹ and n have the same meanings as defined above. In the scheme (i), it is suffice to refer to the method described for example in Organic Synthesis, volume 1, p442 for the method with Na₂SO₃, and to the method described for example in the specification of USP 1,671,257 for the method with SnCl₂. The scheme (ii) can he implemented according to the method described in Chemisch Berichte, 59, pp316-321 (1926) or by the reaction usually at 0°C - 100°C usually in a solvent such as ethanol, usually for 1 hour - 50 hours.

The compound represented by the formula (VI) can be prepared for example according to the following schemes (iii) or (iv): 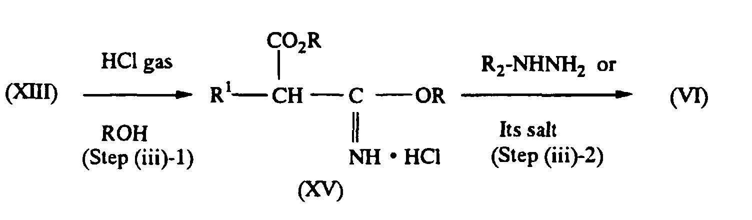 wherein R, R¹, and R² have the same meanings as defined above.

### (Step (iii)-1)

The compound (XV) can be prepared for example by blowing hydrogen chloride gas into the mixture of the compound of the formula (XIII) with the equivalent amount of ROH to a saturation level usually at -5°C to 5°C usually for 30 minutes - 10 hours. A solvent such as diethyl ether may be used, although it is not necessarily essential for the reaction.

### (Step (iii)-2)

The compound (VI) can be prepared by reacting the hydrazine compound represented by the formula R²-NHNH₂ or a salt thereof with the compound (XV) in a solvent, e.g. an alcohol such as methanol and ethanol usually at 5°C - 100°C usually for 1 hour - 48 hours, if necessary after adjusting the mixture to pH of about 7 with a base or acid such as sodium methylate, sodium ethylate and acetic acid. 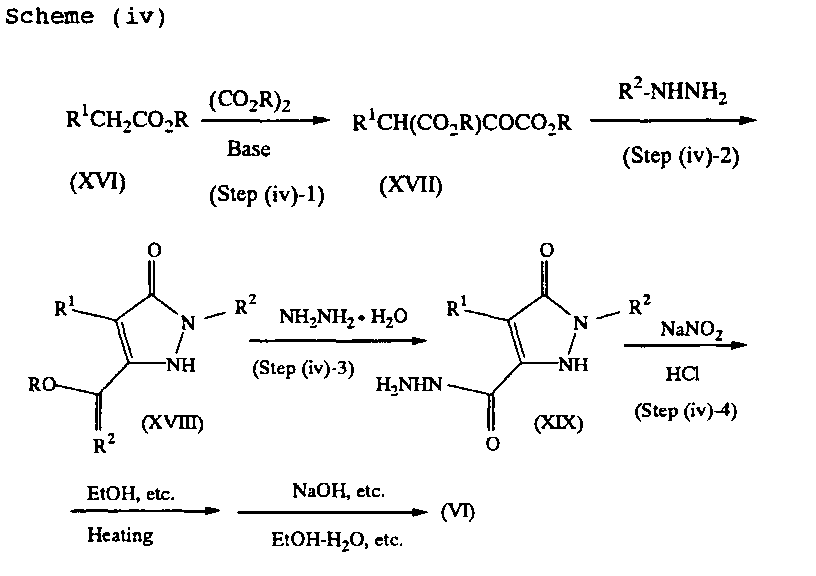 wherein R, R¹, and R² have the same meanings as defined above.

### (Step (iv)-1)

The compound (XVII) can be prepared by reacting the phenylacetic acid ester (XVI) with the compound (CO₂R)₂ in an approximately equivalent amount to the ester usually in the presence of 1.0 equivalent - 1.5 equivalents of a base such as sodium hydride, lithium diisopropylamide and sodium ethylate usually in a solvent such as tetrahydrofuran, N,N-dimethylformamide and ethanol usually at 0°C - 60°C.

### (Step (iv)-2)

The compound (XVIII) can be prepared by reacting the compound (XVII) with the hydrazine compound R²-NHNH₂ or a salt thereof such as the hydrochloride (1.0 equivalent - 1.5 equivalents), if necessary, in a solvent such as ethanol or acetic acid usually at 0°C - 200°C.

### (Step (iv)-3)

The compound (XIX) can be prepared by reacting the compound (XVIII) with hydrazine monohydrate usually in a solvent such as methanol and ethanol usually at 0°C - 120°C usually for 10 minutes - 10 hours.

### (Step (iv)-4)

The compound represented by the formula (IV) can be prepared by (i) acidifying the compound (XIX) usually with 36% hydrochloric acid usually at 0°C - 30°C, adding an aqueous solution of sodium nitrite (1.0 equivalent - 1.5 equivalents) usually at 0°C - 5°C thereto, ii) heating the mixture under refluxing usually for 1 hour - 10 hours, and iii) hydrolyzing the reaction mixture with a base such as sodium hydroxide and potassium hydroxide usually in a solvent such as ethanol and water.

The plant diseases against which the present compound exhibits the controlling effect include rice blast (Pyricularia oryzae), Helmithosporium leaf spot (Cochliobolus miyabeanus), sheath blight (Rhizoctonia solani), powdery mildew (Erysiphe graminis f.sp. hordei, f.sp. tritici), scab (Gibberella zeae), rust (Puccinia striiformis, P. graminis, P. recondita, P. hordei), snow blight (Typhula sp., Micronectriella nivalis), loose smuts (Ustilago tritici, U. nuda), eye spot (Pseudocercosporella herpotrichoides), scald (Rhynchosporium secalis), speckled leaf blotches (Septoria tritici), glume blotch (Leotosphaeria nodorum), net blotch (Pyrenophora teres), citrus fruit melanose (Diaporthe citri), scab (Elsonoe fawcetti), common green mold (Penicillium digitatum, P. italicum), apple tree blossom blight (Sclerotinia mali, Monilinia mali), apple tree canker (Valsa mali), apple tree powdery mildew (Podosphaera leucotricha), Alternaria leaf spot (Alternaria mali), apple tree scab (Venturia inaequalis), pear tree scab (Venturia nashicola), pear tree black spot (Alternari kikuchiana), pear tree rust (Gymnosporangium haraeanum), peach tree rot (Sclerotinia fructigena), blossom blight (Monilinia fructigena), peach tree brown rot (Sclerotinia cinerea, Monilinia fructicola), peach tree scab (Cladosporium carpophilum), peach tree Phomopsis (Phomopsis sp.), vine anthracnose (Elsinoe ampelina), vine ripe rot (Glomerella cingulate), vine powdery mildew (Uncinula nacator), vine rust (Phakopsora ampelopsidis), persimmon tree anthracnose (Gloeosporium kaki), persimmon tree angula leaf spot (Cercospora kaki, Mycosphaerella nawae), cucurbitaceous anthracnose (Colletotrichum lagenarium), cucurbitaceous powdery mildew (Sphaerotheca fuliginea), cucurbitaceous gummy stem blight (Mycosphaerella melonis), tomato early blight (Alternaria solani), tomato leaf mold (Cladosporium fulvum), eggplant brown spot (Phomopsis vexans), egg plant powdery mildew (Erysiphe cichoracearum), cruciferae vegetable gray leaf spot (Alternaria japonica), cruciferae vegetable white spot (Cercosporella brassicae), Welsh onion rust (Pucciniaallii), soybean purple (Cercospora kikuchii), soybean Sphaceloma scab (Elsinoe glycines), soybean pod and stem blight (Diaporthe phaseolorum var. sajae), sidney bean anthracnose (Colletotrichum lindemuthianum), peanut leaf spot (Mycosphaerella personnatum), Cercospora leaf spot (Cercospora arachidicola), pea powdery mildew (Erysiphe pisi), potato early blight (Alternaria solani), strawberry powdery mildew (Sphaerotheca humuli), tea plant net blister blight (Exobasidium reticulatum), tea plant scab (Elsinoe leucospila), tobacco brown spot (Alternaria longipes), tobacco powdery mildew (Erysiphe cichoracearum), tobacco anthracnose (Colletotrichum tabacum), leaf spot (Cercospora berticola), rose black spot (Piplocarpon rosae), rose powdery mildew (Sphaerotheca pannosa), chrysanthemum leaf blight (Sentoria chrysanthemi-indici), chrysanthemum rust (Puccinia horiana), gray mold of various crops products (Botrytis cinerea), Botrytis diseases of various crops (Botrytis ssp.), Scherotixia rots of various crops (Sclerotinia sclerotiorum), and the like. Particularly, the present compound shows an excellent protection effect against powdery mildew in wheat.

When the present compound is used as an effective ingredient of a plant disease controlling agent, it is usually used in the form of a formulation such as emulsifiable concentrate, wettable powder, suspension concentrate, granule, dust and solution which is obtained usually by blending the present compound usually with the other formulation adjuvants such as solid support, liquid support and surface active agent, although it may be used as such without the addition of any of the other ingredients.

The present compound is contained as the effective ingredient of these formulations usually in a proportion of 0.1 - 99%, preferably 1 - 80% by weight.

The solid support includes fine powder or granules such as kaolin clay, attapulgite clay, bentonite, acid clay, pyrophillite, talc, diatomaceous earth, calcite, walnut shell powder, urea, ammonium sulfate and synthetic silicon hydroxide. The liquid support includes aromatic hydrocarbons such as xylene and methylnaphthalene, alcohols such as isopropanol, ethylene glycol and cellosolve, ketones such as acetone, cyclohexanone and isophorone, vegetable oils such as soybean oil and cotton seed oil, dimethylsulfoxide, acetonitrile and water. The surface active agent used for emulsification, dispersion or wetting includes anionic surface active agents such as alkyl sulfate ester, alkyl(aryl)sulfonate, dialkylsulfosuccinate and polyoxyethylene-alkyletherphosphate, nonionic surface active agents such as polyoxyethylenealkylether, polyoxyethylenealkylarylether, polyoxyethylenepolyoxypropylene block copolymer, sorbitan fatty acid ester and polyoxyethylenesorbitan fatty acid ester. The adjuvant for the formulation includes lignin sulfonate, alginate, polyvinyl alcohol, gum arabic, CMC (carboxymethyl-cellulose), PAP (acidic isopropyl phosphate), and the like.

These formulations are used in various forms such as spraying directly or in an aqueous dilution on stems or leaves, treatment of seeds, dusting powder or granules on soil, and blending, if necessary. Also, these formulations may be mixed with the other plant disease controlling agents to enhance the disease controlling effect. In addition, these formulations may be used as the mixtures with insecticide, acaricide, nematicide, herbicide, plant growth regulator, fertilizer, or soil conditioner.

The other plant disease controlling agents which can be blended include for example azole type bacteriocidal compounds such as propiconazole, triadinenol, prochloraz, penconazole, tebuconazole, flusilazole, diniconazole, bromuconazole, epoxyconazole, difenoconazole, cyproconazole, metconazole, triflumizole, tetraconazole, myclobutanil, fenbuconazole, hexaconazole, fluquinconazole, triticonazole, bitertanol, imazalil and flutriafol, cyclic amine type bacteriocidal compounds such as fenpropimorph, tridemorph and fenpropidin, benzimidazole type bacteriocidal compounds such as carbendazim, benomyl, thiabendazole and thiophanate-methyl, procymidone, cyprodinil, pyrimethanil, diethofencarb, thiuram, fluazinam, mancozeb, iprodione, vinclozolin, chlorothalonil, captan, mepanipyrim, fenpiclonil, fludioxonil, dichlorfluanid, folpet, methyl methoxyimino-α-(o-tolyloxyl)-o-tolylacetate (BAS490F), methyl (E)-{2-[6-(2-cyanophenoxy)pyrimidin-4-yloxy]phenyl}-3-methoxyacrylate (ICIA5504) and N-methyl-α-methoxyimino-2-[(2,5-dimethylphenoxy)methyl]phenylacetamide.

In this respect, the present compound can be used as an effective ingredient of plant disease controlling agents for paddy field, upland field, orchard, tea field, pasture, lawn, and the like.

When the present compound is used as an effective ingredient of plant disease controlling agents, it is applied generally in an amount of 0.001 g - 100 g/are, preferably 0.01 g - 10 g/are; when the emulsifiable concentrate, the wettable powder, the suspension concentrate, the solution or the like is used as an aqueous dilution, it is applied generally in a concentration of 0.001% - 0.1%. The granules and powder are directly applied without dilution.

In the case of seed treatment, the present compound can be applied generally in an amount of 0.001 g - 100 g, preferably 0.01 g of 10 g of the effective ingredient per 1 kg of seed.

The present invention is further described in detail below with reference to Preparation Examples, Formulation Examples, and Test Examples without limit thereto.

First, the preparation examples of the present compounds are illustrated.

### Preparation Example 1

A mixture of 5-amino-4-(3-chlorophenyl)-1-(pyridin-2-yl)pyrazoline-3-one (1.4 g), 2-iodopropane (1.6 g), potassium carbonate (2.0 g), and ethanol (30 ml) was stirred at room temperature for 24 hours. The reaction mixture was poured into ice-water and extracted with ethyl acetate. The organic layer was dried over anhydrous magnesium sulfate, and then concentrated under reduced pressure. The residue was subjected to column chromatography (eluent; hexane : ethyl acetate = 1 : 1) to give 0.42 g of 5-amino-4-(3-chlorophenyl)-2-isopropyl-1-(pyridin-2-yl)pyrazolin-3-one (the present compound a-20).
Melting point: 130.5°C.
¹H-NMR (CDCl₃/TMS) δ (ppm): 8.44 (dd, 1H), 7.82 (td, 1H), 7.61 (brs, 1H), 7.50 (d, 1H), 7.24 - 7.36 (m, 2H), 7.09 - 7.11 (m, 2H), 6.41 (brs, 2H), 3.81 (m, 1H), 1.34 (d, 6H).

### Preparation Example 2

To a mixture of 5-amino-4-(3-chlorophenyl)-1-(pyrimidin-2-yl)pyrazolin-3-one (1.5 g), potassium carbonate (2.2 g) and ethanol (30 ml) was added 1-methylpropyl iodide, and the resulting mixture was stirred at room temperature for 72 hours. The reaction mixture was poured into water, and extracted with ethyl acetate. The organic layer was dried over anhydrous magnesium sulfate, and then concentrated under reduced pressure. The residue was subjected to column chromatography (eluent; hexane : ethyl acetate = 1 : 1) to give 0.4 g of 5-amino-4-(3-chlorophenyl)-2-(1-methylpropyl)-1-(pyrimidin-2-yl)pyrazolin-3-one (the present compound b-3).
Melting point: 117.9°C.
¹H-NMR (CDCl₃/TMS) δ (ppm): 8.69 (d, 2H), 7.64 (t, 1H), 7.51 (dd, 1H), 7.32 (t, 1H), 7.08 - 7.18 (m, 2H), 6.62 (brs, 2H), 3.88 (m, 1H), 2.19 (m, 1H), 1.88 (m, 1H), 1.44 (d, 3H), 1.01 (t, 3H).

### Preparation Example 3

To a mixture of 5-amino-2,4-bis(2-methylphenyl)pyrazolin-3-one (0.1 g), potassium carbonate (0.08 g) and ethanol (3 ml) was added 2-chloropyrimidine, and the resulting mixture was stirred at room temperature for 24 hours. The reaction mixture was poured into water, and extracted with ethyl acetate. The organic layer was dried over anhydrous magnesium sulfate, and then concentrated under reduced pressure. The residue was subjected to column chromatography (eluent; hexane : ethyl acetate = 1 : 1) to give 0.02 g of 5-amino-2,4-bis(2-methylphenyl)-2-(pyrimidin-2-yl)pyrazolin-3-one (the present compound b-4).
¹H-NMR (CDCl₃/TMS) δ (ppm): 8.47 (d, 2H), 7.04 - 7.42 (m, 8H), 6.95 (t, 1H), 6.37 (brs, 2H), 3.39 (s, 3H), 2.61 (s, 3H).

### Preparation Example 4

5-amino-2-(2-methylpropyl)-4-(1-naphthyl)-1-(pyridin-2-yl)-pyrazolin-3-one (0.3 g) and N,N-dimethylformamide dimethyl acetal (1 ml) were stirred at 130°C for 3 hours. The reaction mixture was cooled to room temperature, and extracted after addition of ethyl acetate and water. The organic layer was dried over anhydrous magnesium sulfate, and then concentrated under reduced pressure. The residue was subjected to column chromatography (eluent; hexane : ethyl acetate = 1 : 1) to give 0.2 g of 5-(N,N-dimethylaminomethylidenamino)-2-(2-methylpropyl)-4-(1-naphthyl)-1-(pyridin-2-yl)pyrazolin-3-one (the present compound d-16).
¹H-NMR (CDCl₃/TMS) δ (ppm): 8.47 (d, 2H), 7.88 (m, 1H), 7.78 (m, 3H), 7.59 (d, 1H), 7.39 - 7.45 (m, 4H), 7.14 (dd, 1H), 7.03 (s, 1H), 4.06 (m, 1H), 2.57 (s, 3H), 2.25 (s, 3H), 1.90 (m, 1H), 1.50 (1H, m), 1.18 (d, 3H), 0.86 (t, 3H).

### Preparation Example 5

To a solution of sodium hydride (0.06 g) in N,N-dimethylformamide (2 ml) was added 5-amino-2-(2-methylpropyl)-4-(1-naphthyl)-1-(pyridin-2-yl)pyrazolin-3-one (0.5 g), and the mixture was stirred at 100°C for 1 hour and then cooled to 0°C. After acetyl chloride (0.11 g) was added dropwise at the same temperature, the mixture was stirred at 90°C for 2 hours. The reaction mixture was poured into water, and extracted with ethyl acetate. The organic layer was dried over anhydrous magnesium sulfate, and then concentrated under reduced pressure. The residue was subjected to column chromatography (eluent; hexane : ethyl acetate = 1 : 1) to give 0.3 g of 5-acetylamino-2-(2-methylpropyl)-4-(1-naphthyl)-1-(pyridin-2-yl)pyrazolin-3-one (the present compound d-14).
¹H-NMR (CDCl₃/TMS) δ (ppm): 8.46 (dd, 1H), 7.85 (m, 1H), 7.67 - 7.81 (m, 3H), 7.30 - 7.55 (m, 4H), 7.22 (t, 3H), 4.11 (m, 1H), 1.89 (m, 1H), 1.63 (m, 1H), 1.49 (brs, 3H), 1.22 (d, 3H), 0.86 (t, 3H).

### Preparation Example 6

To a mixture of 5-amino-4-(3-chlorophenyl)-2-isopropyl-1-(pyridin-2-yl)-pyrazolin-3-one (0.6 g), 45% aqueous solution of sodium hydroxide (0.7 ml), tetra-n-butylammonium bromide (10 mg) and dichloromethane (3 ml) was added diethyl sulfate (0.56 g), and the mixture was stirred at room temperature for 15 hours. The reaction mixture was diluted with dichloromethane and water for extraction. The organic layer was dried over anhydrous magnesium sulfate, and then concentrated under reduced pressure. The residue was subjected to column chromatography (eluent; hexane : ethyl acetate = 2 : 1) to give 4-(3-chlorophenyl)-5-(N-ethylamino)-2-isopropyl-1-(pyridin-2-yl)pyrazolin-3-one (the present compound d-4) and 4-(3-chlorophenyl)-5-(N,N-diethylamino)-2-isopropyl-1-(pyridin-2-yl)pyrazolin-3-one (the present compound d-3) in a yield of 0.33 g and 0.08, respectively.

### The present compound d-4

¹H-NMR (CDCl₃/TMS) δ (ppm): 8.44 (dd, 1H), 7.83 (td, 1H), 7.60 (brt, 1H), 7.10 - 7.48 (m, 5H), 3.82 (m, 1H), 2.93 (m, 2H), 1.32 (d, 6H), 0.90 (t, 3H).

### The present compound d-3

¹H-NMR (CDCl₃/TMS) δ (ppm): 8.47 (dd, 1H), 7.72 (td, 1H), 7.41 (brs, 1H), 7.32 (d, 1H), 7.15 - 7.28 (m, 2H), 7.10 (brd, 2H), 4.31 (m, 1H), 3.07 (q, 4H), 1.07 (d, 6H), 0.76 (t, 6H).

Next, examples of the present compounds are shown in Tables 3 - 6. In this connection, in these tables, Et represents an ethyl group, nPr represents a propyl group, iPr represents an isopropyl group, nBu represents a butyl group, secBu represents a sec-butyl group, iBu represents an isobutyl group, Ph represents a phenyl group, and Py represents a pyridyl group, respectively.

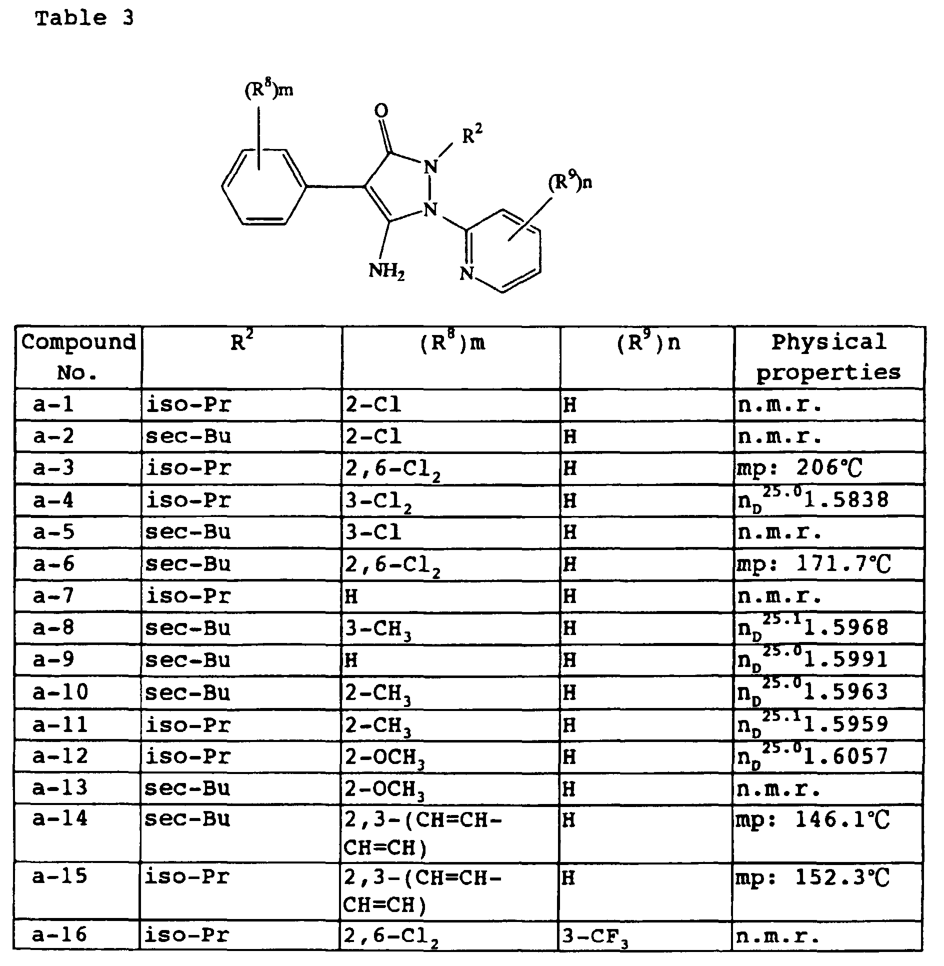

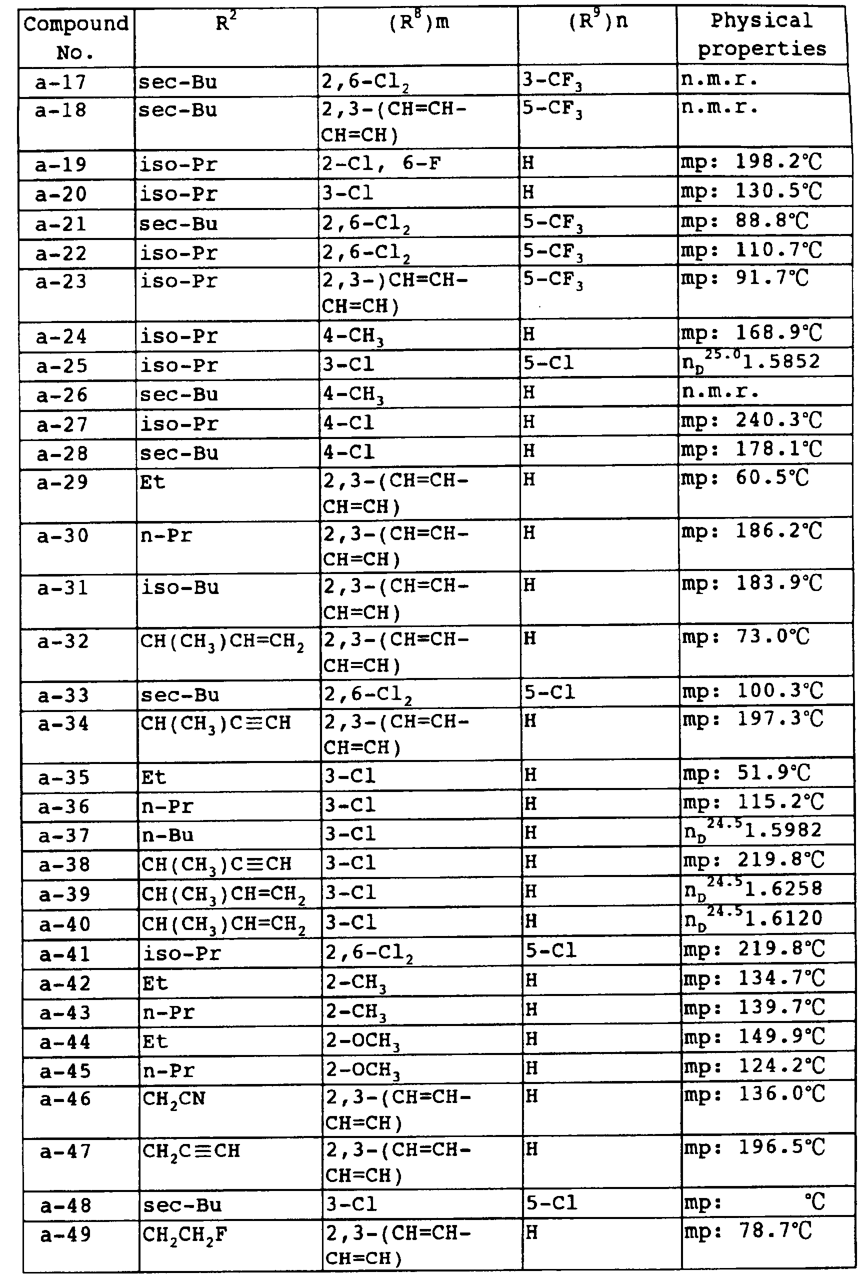

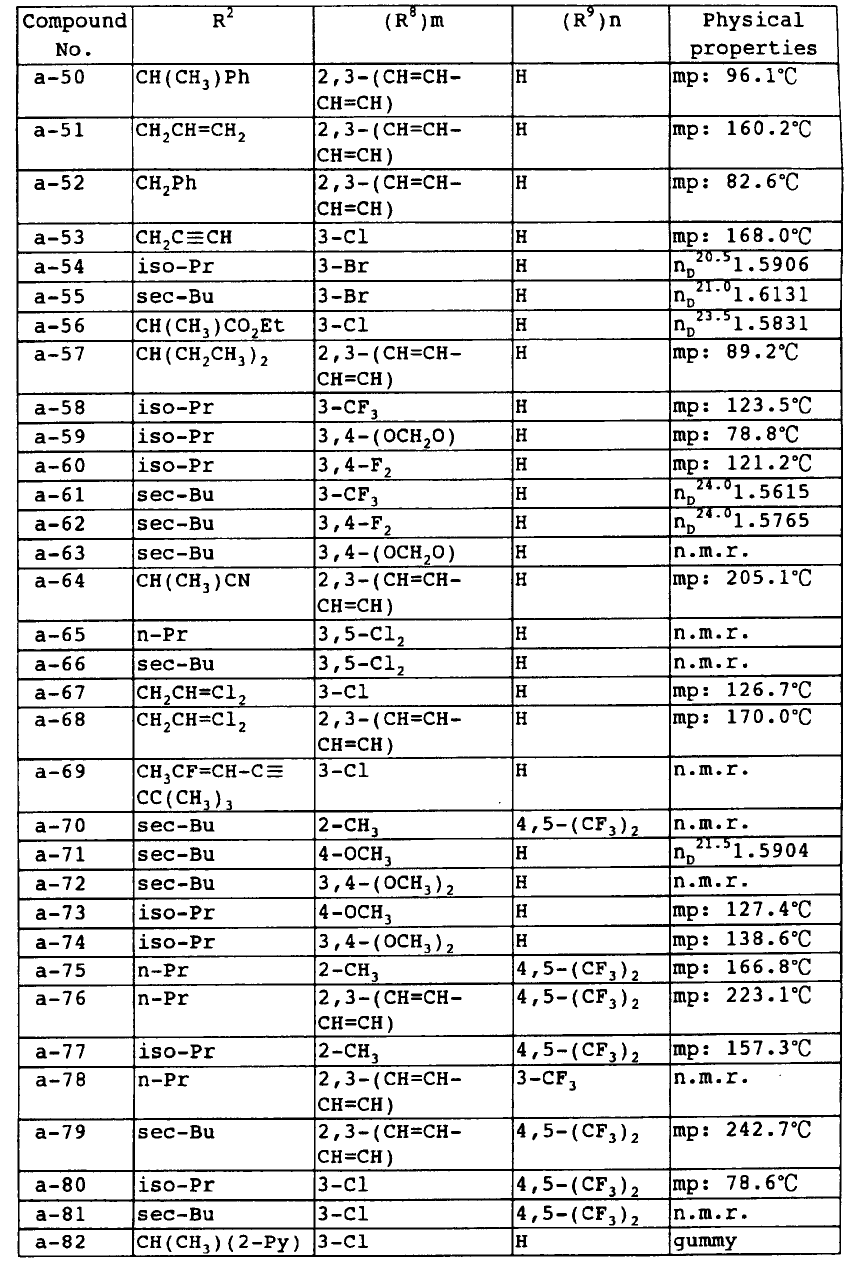

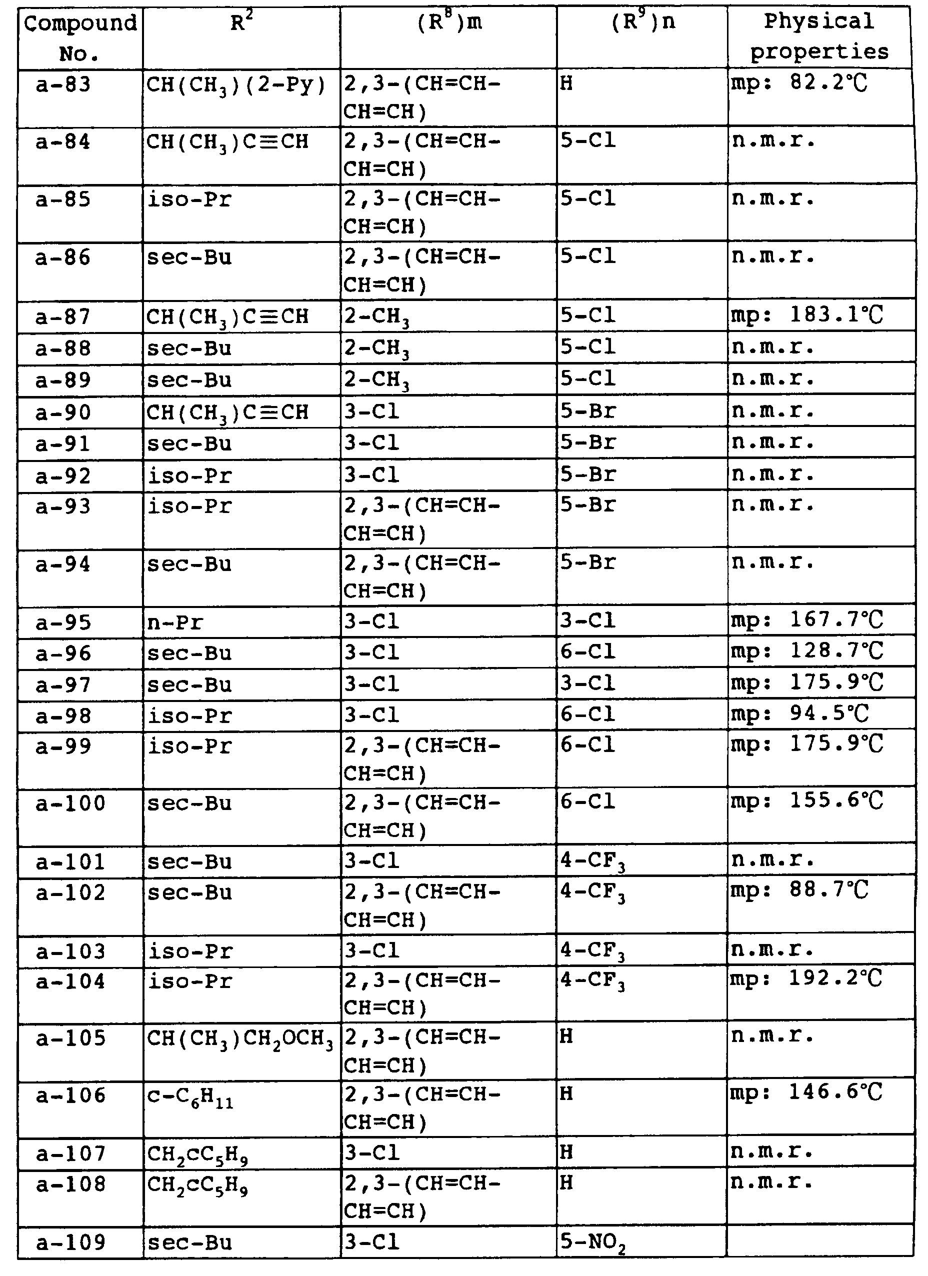

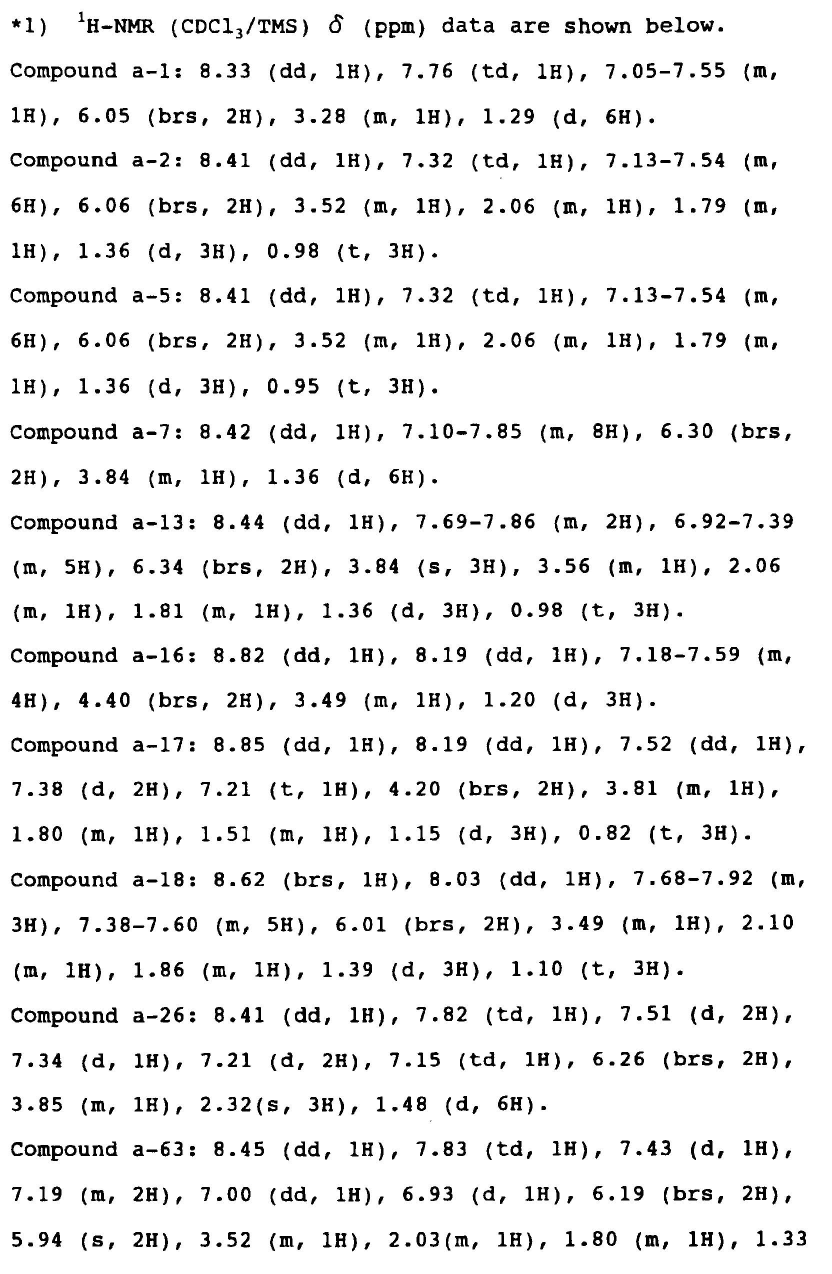

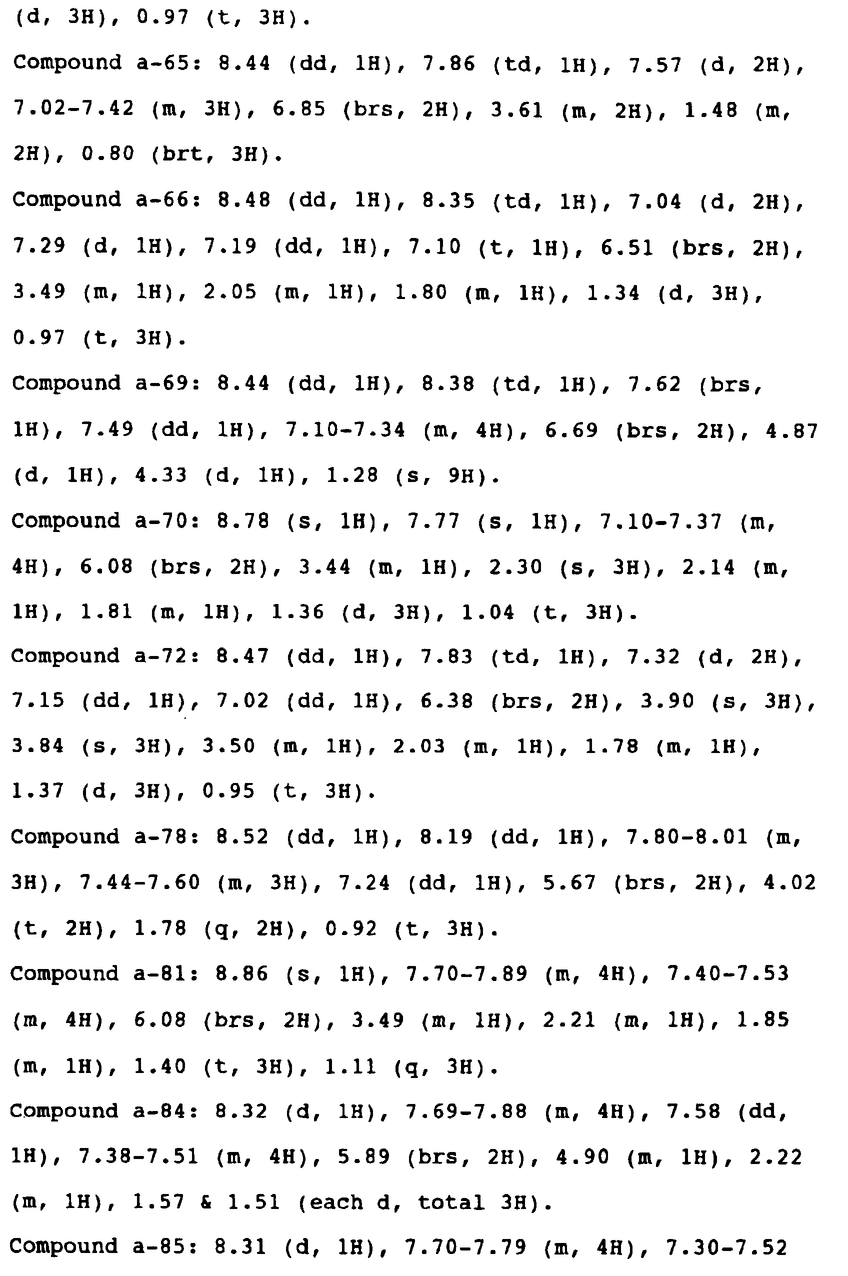

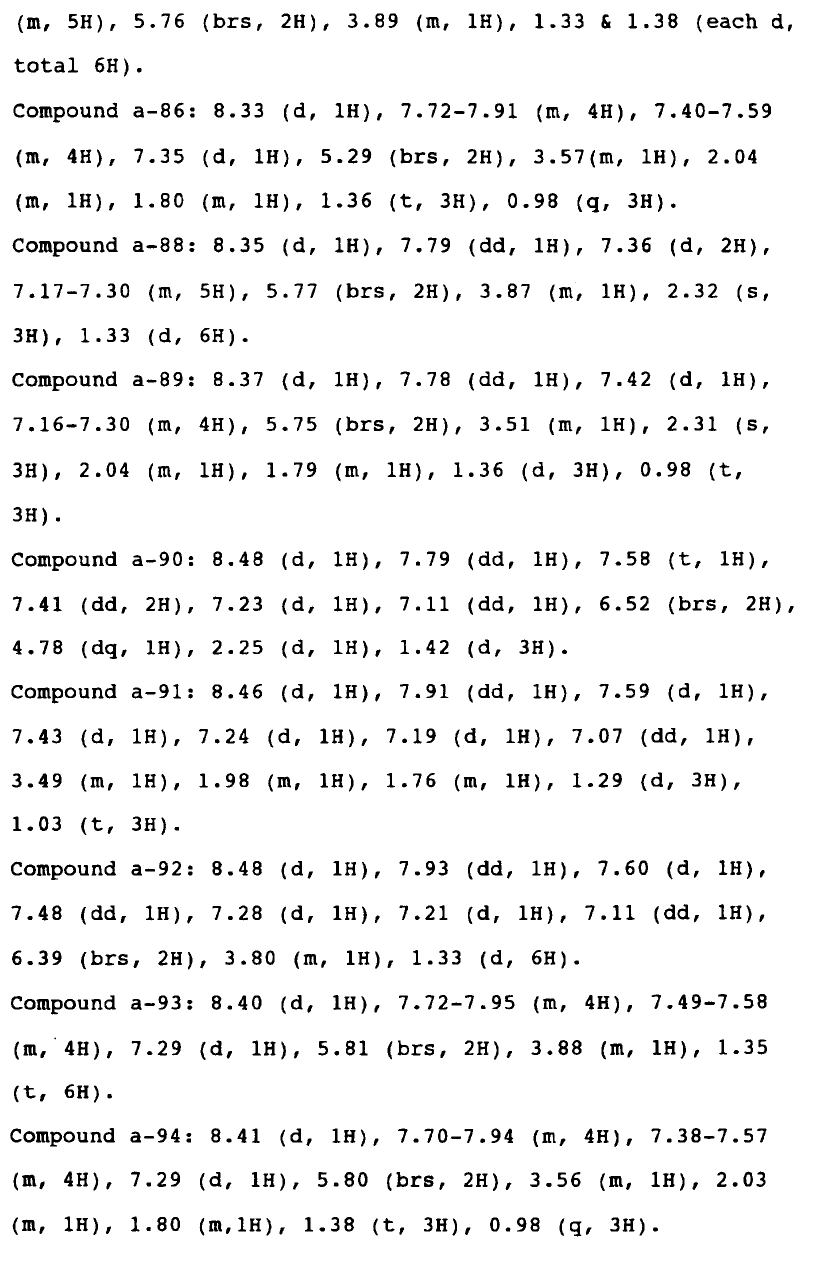

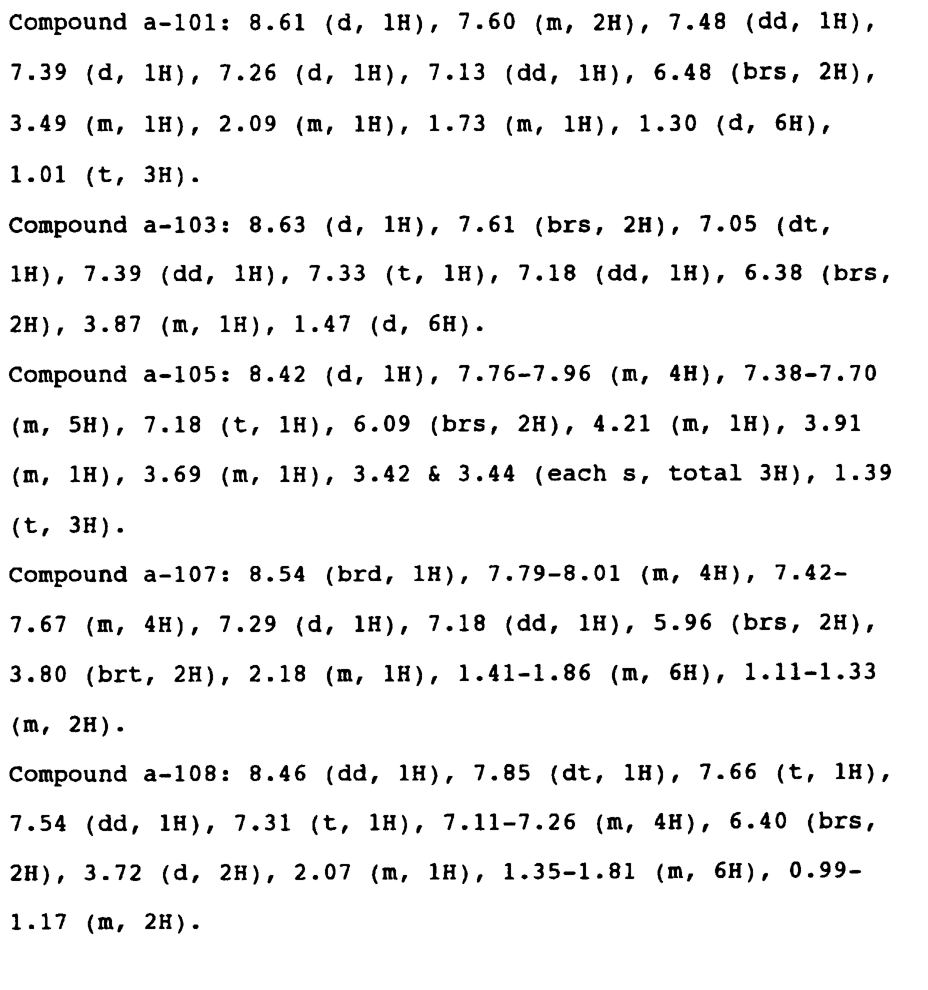

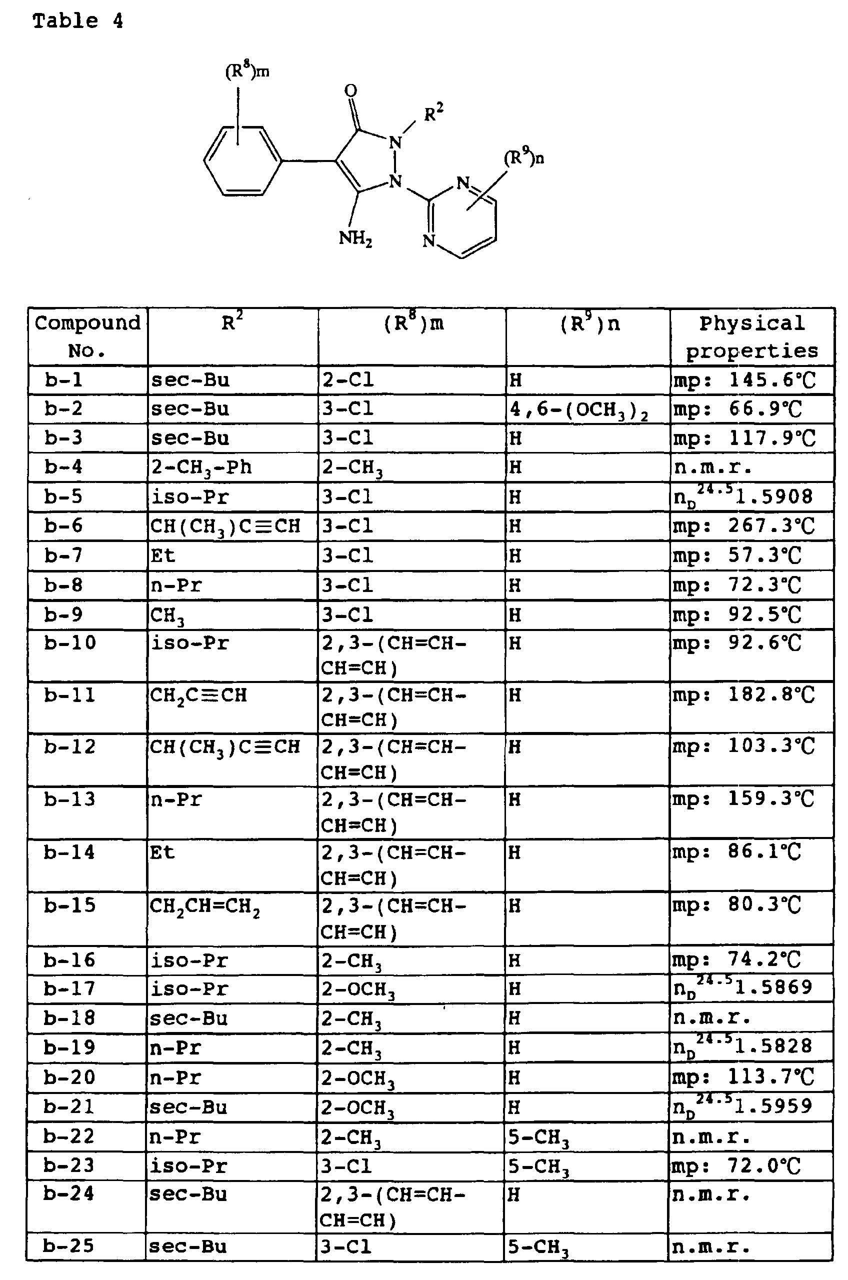

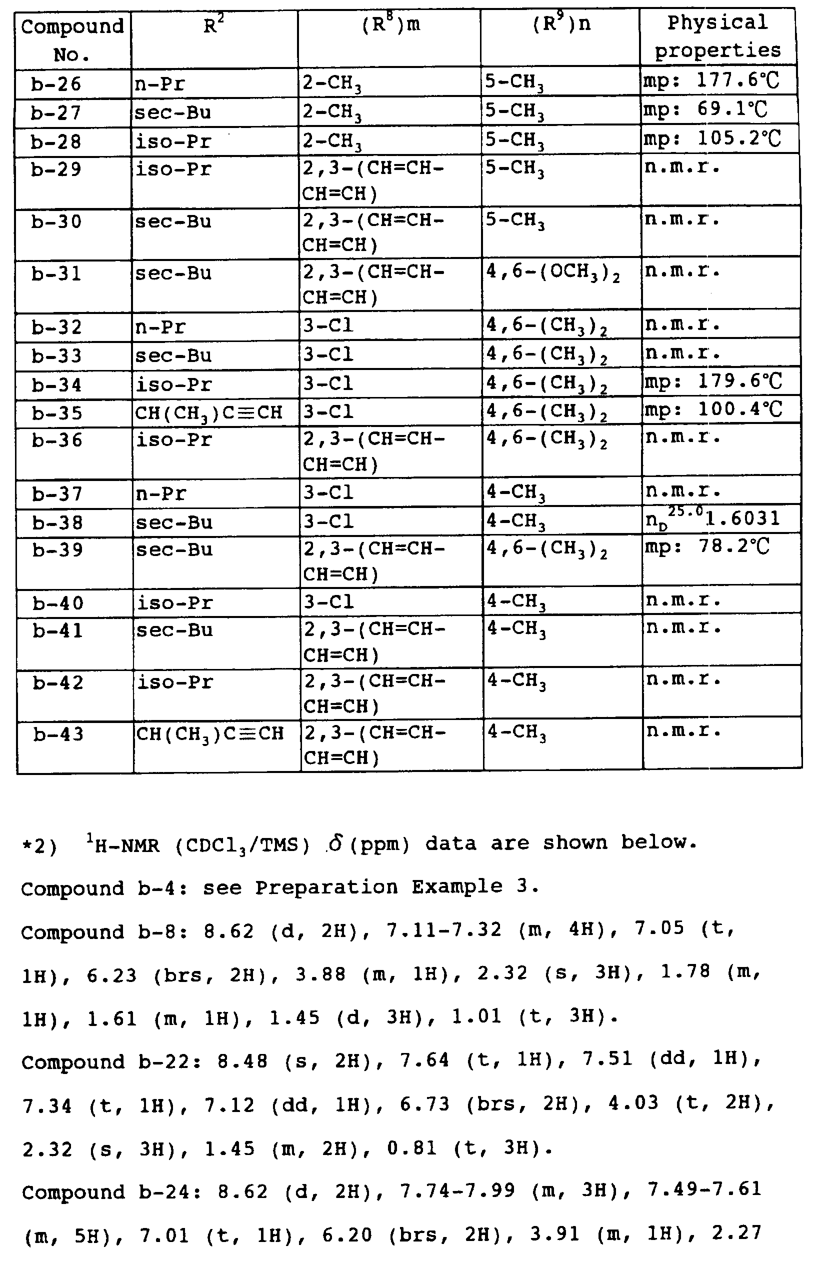

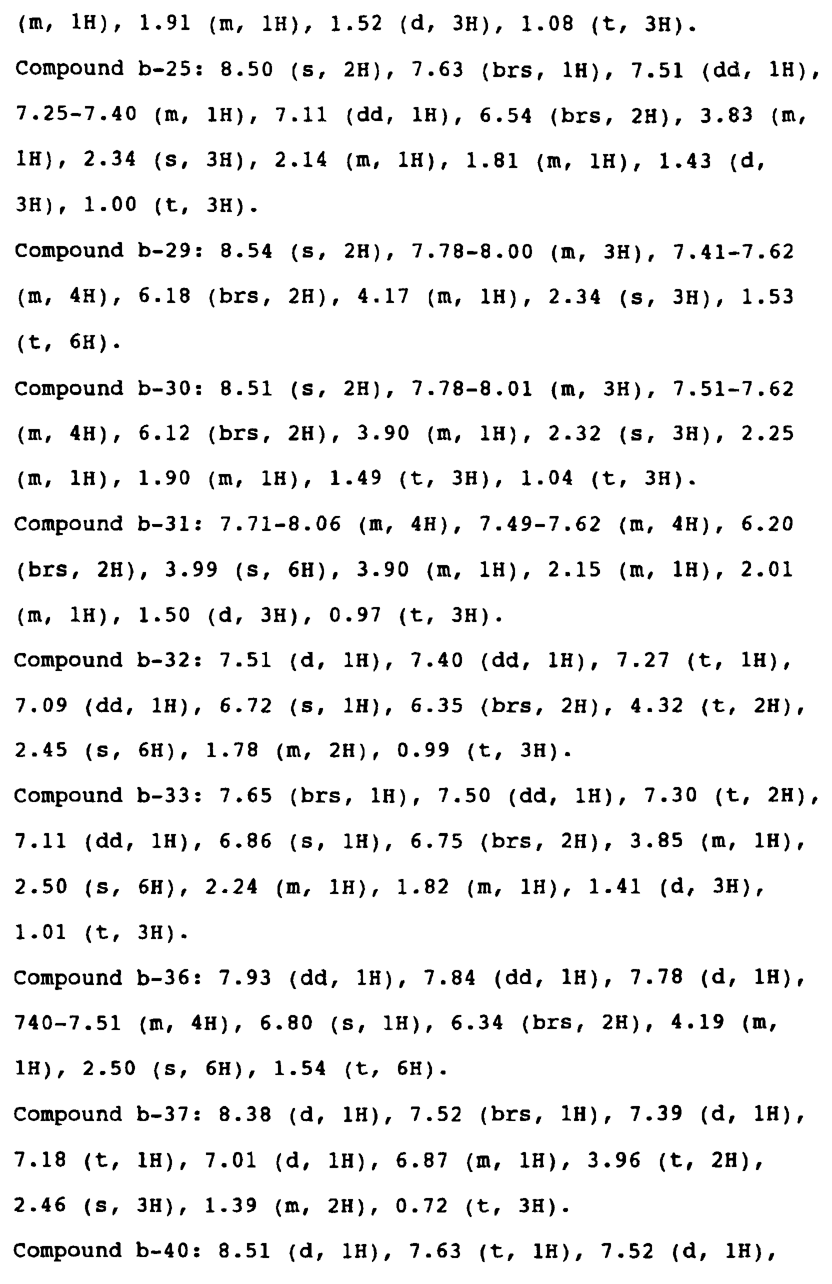

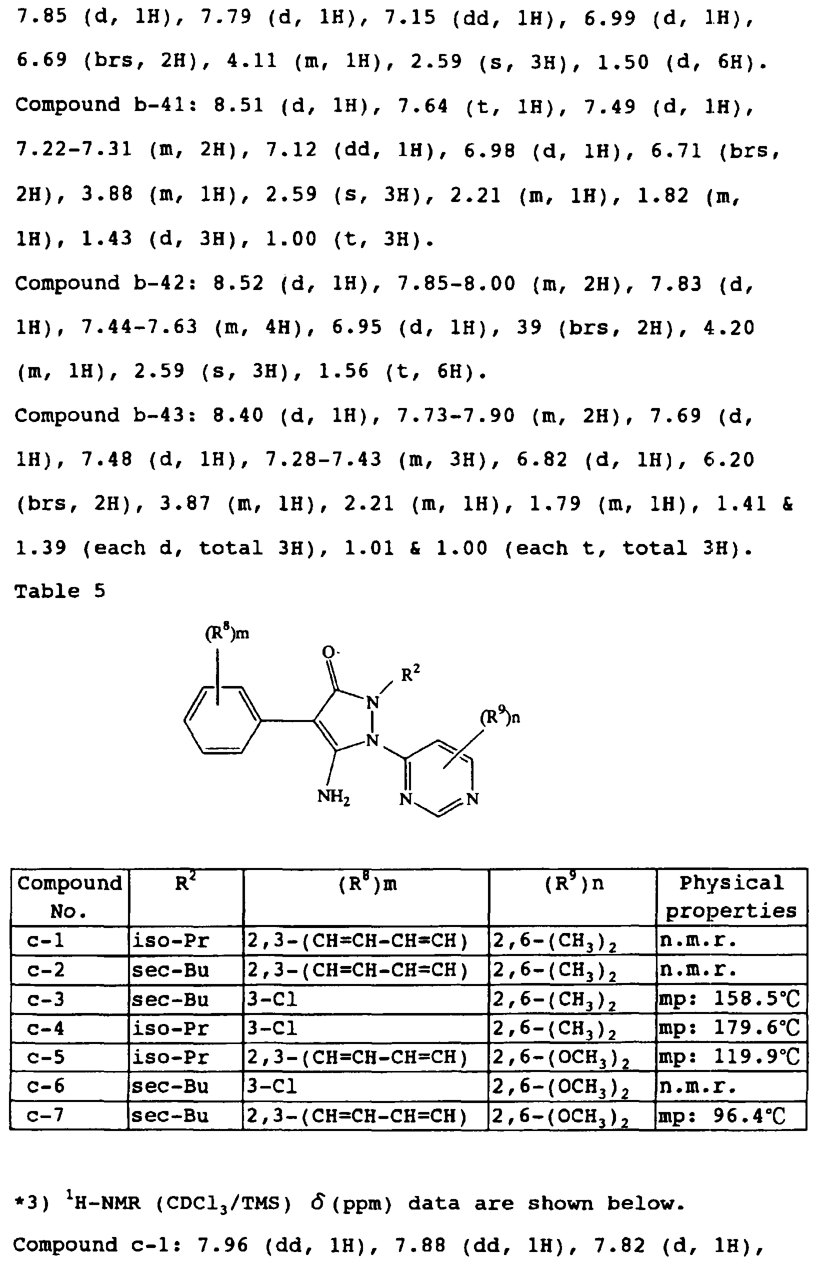

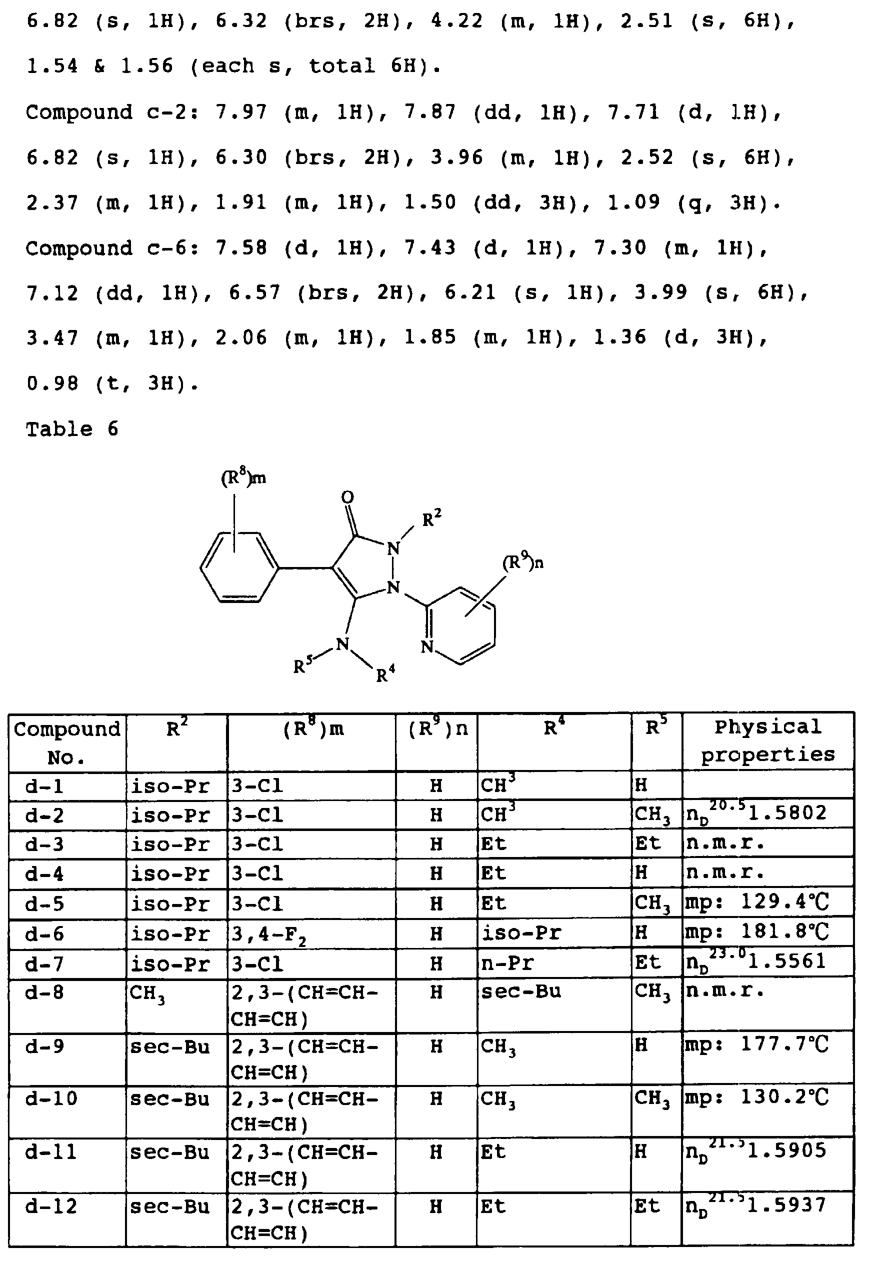

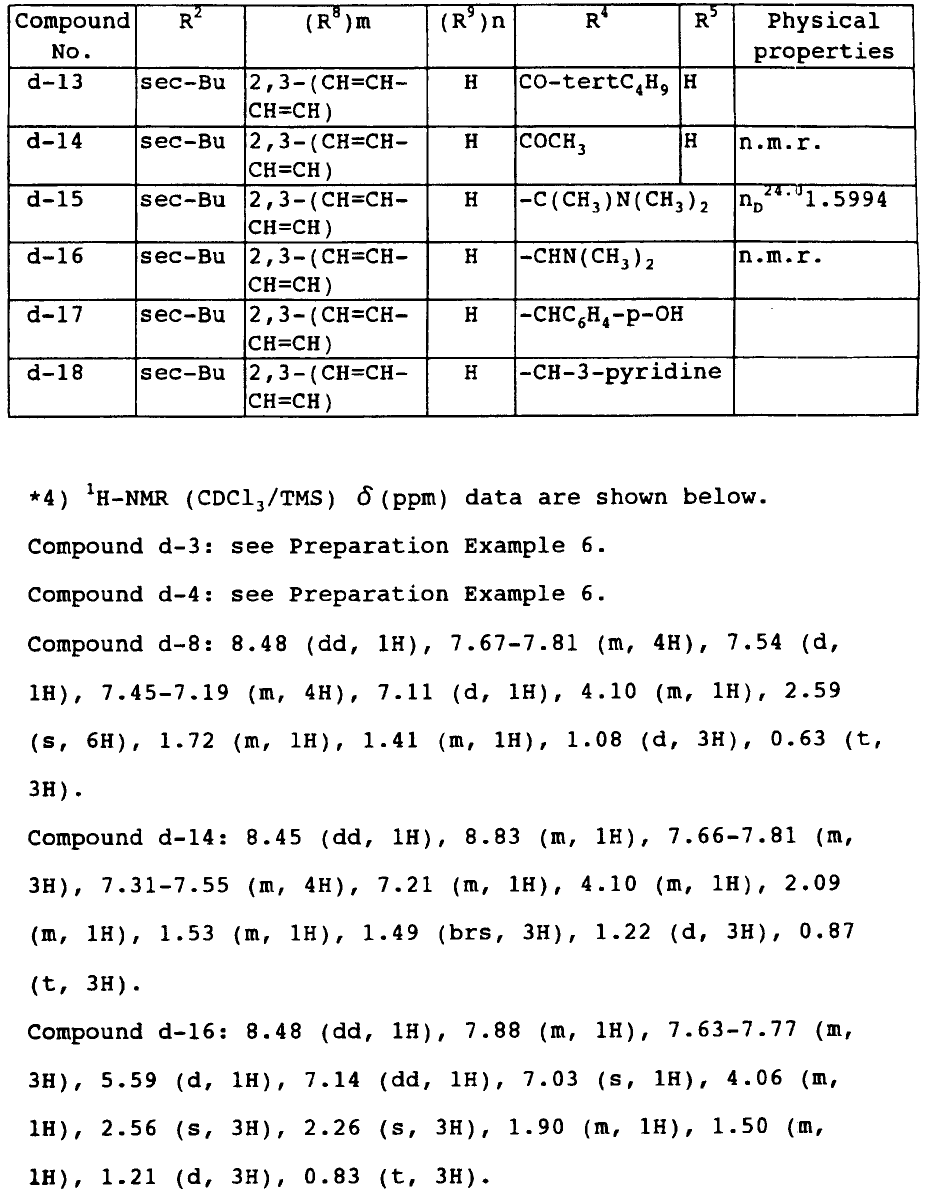

Next, preparation examples of the aminopyrazoline compounds represented by the formula (II) are described below.

### Preparation Example 1 of Intermediate Compound

A mixture of 2-hydrazinopyridine (2.43 g) and ethyl 3-chlorophenylcyanoacetate (5.0 g) was heated at 170°C for 3 hours, and then cooled to room temperature. The crystallized product was collected with hexane, washed with hexane to give 5-amino-4-(3-chlorophenyl)-1-(pyridin-2-yl)pyrazolin-3-one (5.8 g). Melting point: 265.7°C

### Preparation Example 2 of Intermediate Compound

Hydrazine monohydrate (43.5 g) was added to a solution of 2-chloropyrimidine (33 g) in ethanol, and the mixture was stirred at room temperature for 15 hours. The reaction mixture was concentrated under reduced pressure, and the crystalline product thus obtained was recrystallized from ethyl acetate (50 ml) to give 2-hydrazinopyrimidine (20 g). Melting point 109.5°C.
¹H-NMR (CDCl₃/DMSO-d6/TMS) δ (ppm): 8.34 (d, 2H), 6.38 (brs, 1H), 6.62 (t, 1H), 3.98 (brs, 2H).

A mixture of 2-hydrazinopyrimidine (1.0 g) and ethyl 2-chlorophenylacetate (2.0 g) were heated at 170°C for 5 hours, and then cooled to room temperature. The precipitated crystalline product was collected with hexane, and washed with hexane to give 5-amino-4-(2-chlorophenyl)-1-(pyrimidin-2-yl)pyrazolin-3-one (2.2 g).
Melting point 102.3°C.

Next, examples of the aminopyrazoline compounds represented by the formula (II) were listed in Tables 7, 8 and 9.

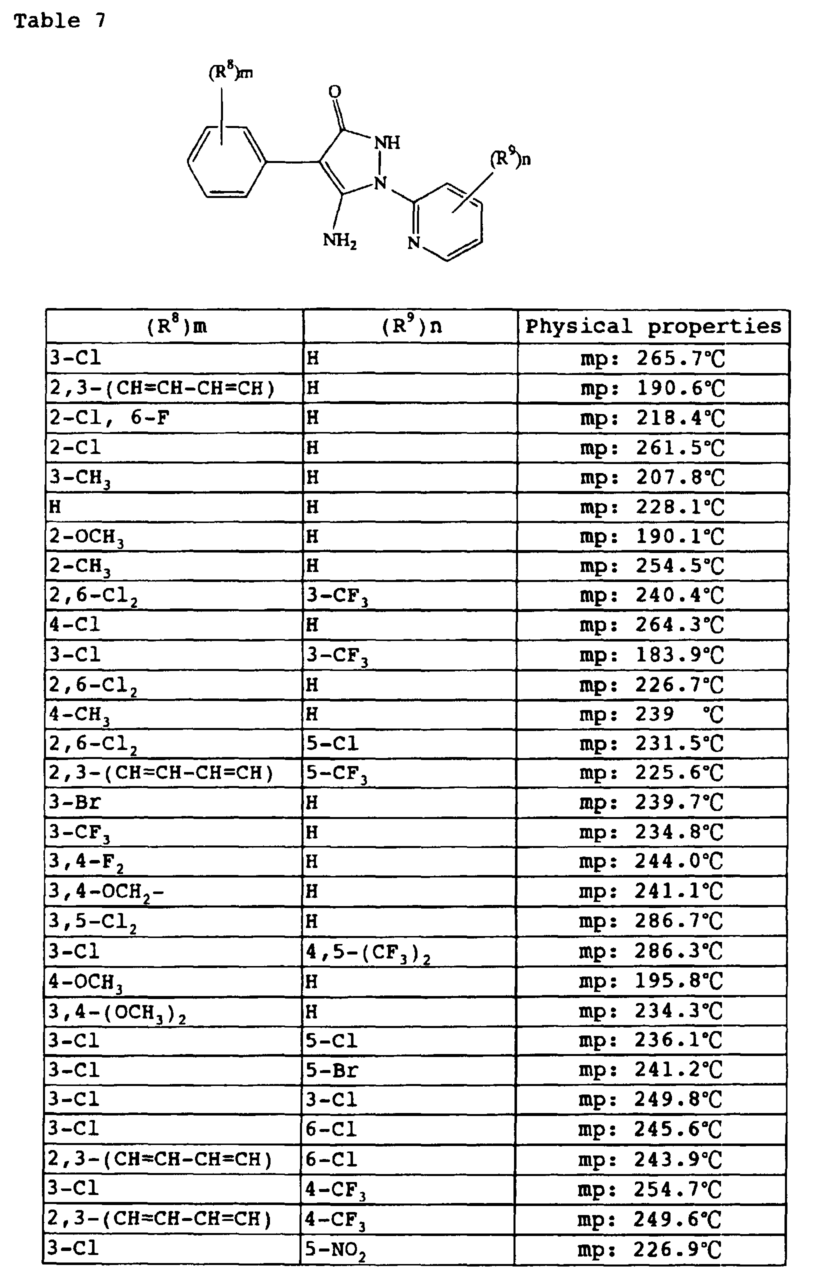

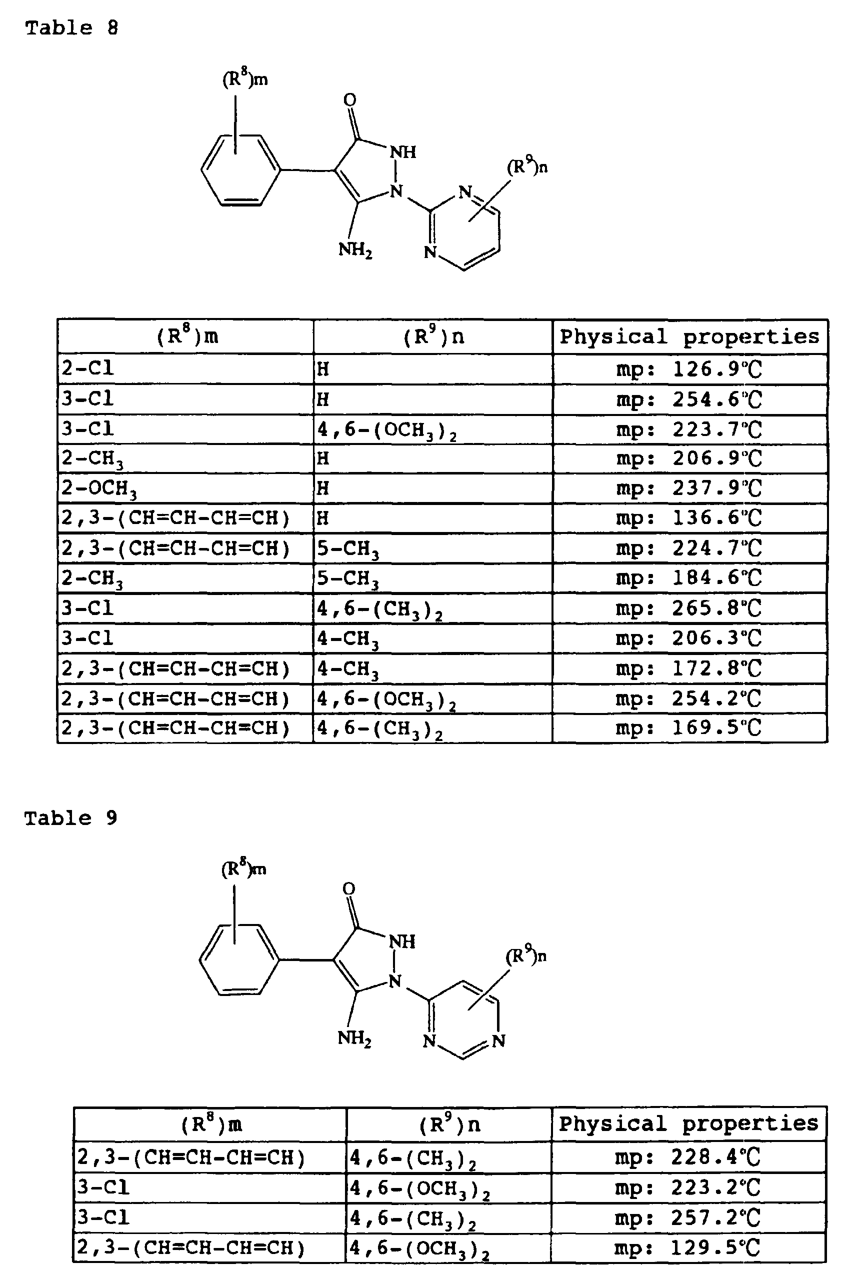

Next, the preparation examples of the compounds represented by the formula (VI) are illustrated.

### Referential Preparation Example 1

To a solution of sodium ethoxide (2.6 g) in ethanol was added diethyl oxalate (8.03 g), followed by ethyl 2-methylphenylacetate (8.9 g), and the mixture was stirred at 50°C for 30 minutes. The reaction mixture was concentrated under reduced pressure. The residue was diluted with acetic acid (100 ml) 2-methylphenyl-hydrazine (9.52 g), and the mixture was heated under reflux for 48 hours. The reaction mixture was concentrated under reduced pressure. The residue was diluted with water, extracted with ethyl acetate, and the organic layer was concentrated. The residue was subjected to chromatography to give 5-ethoxycarbonyl-2,4-bis(2-methylphenyl)-pyrazolin-3-one (8.12 g). Melting point: 173.9°C.

To 5-ethoxycarbonyl-2,4-bis(2-methylphenyl)-pyrazolin-3-one (4.55 g) was added hydrazine monohydrate (20 ml), and the mixture was heated under reflux. The residue was subjected to chromatography to give 5-hydrazinocarbonyl-2,4-bis(2-methylphenyl)-pyrazolin-3-one (3.96 g).
¹H-NMR (DMSO-d6/TMS) δ (ppm): 9.08 (brs, 1H), 7.1-7.5 (m, 8H), 2.17 (s, 3H), 2.14 (s, 3H).

To a solution of 5-hydrazinocarbonyl-2,4-bis(2-methylphenyl)-pyrazolin-3-one (3.66 g) and ethanol (60 ml) in water (20 ml) was added 36% hydrochloric acid (2.5 ml) at 0°C, and the mixture was stirred for 1 hour. A solution of sodium nitrite (1.27 g) in water (13 ml) was then added dropwise at a temperature of 5°C or less, and the mixture was stirred at 0°C for 1 hour. The reaction mixture was concentrated under reduced pressure, and the resulting residue was diluted with ethanol (150 ml) and heated under reflux for 3 hours. The reaction mixture was concentrated under reduced pressure, and the residue thus obtained was subjected to chromatography to give 5-ethoxycarbonylamino-2,4-bis(2-methylphenyl)-pyrazolin-3-one (2.86 g).
¹H-NMR (DMSO-d6/TMS) δ (ppm): 9.00 (brs, 1H), 7.14-7.3 (m, 8H), 4.11 (q, 2H), 1.07 (t, 3H).

To a solution of 5-ethoxycarbonylamono-2,4-bis(2-methylphenyl)-pyrazolin-3-one (2.56 g) and ethanol (30 ml) in water (60 ml) was added sodium hydroxide (6.0 g), and the mixture was heated under reflux for 8 hours. The reaction mixture was cooled to room temperature, acetic acid (15 ml) was added, and then concentrated under reduced pressure. The resulting residue was extracted with ethyl acetate, and the organic layer was dried over anhydrous magnesium sulfate. The residue obtained by concentration under reduced pressure was subjected to chromatography to give 5-amino-2,4-bis(2-methylphenyl)-pyrazolin-3-one (2.1 g). Melting point: 179.9°C.

Formulation examples are illustrated below. In this respect, the present compounds are represented by the compound numbers listed in Tables 3 - 5. Also, parts are based on weight.

### Formulation Example 1

Each of the present compounds a-1 to a-109, b-1 to b-43, c-1 to c-7, and d-1 to d-18 in an amount of 50 parts, 3 parts of calcium ligninsulfonate, 2 parts of sodium laurylsulfate, and 45 parts synthetic hydrous silicon oxide are blended well with grinding to give the corresponding wettable powder.

### Formulation Example 2

Each of the present compounds a-1 to a-109, b-1 to b-43, c-1 to c-7, and d-1 to d-18 in an amount of 10 parts, 14 parts of polyoxy-ethylenestylphenylether, 6 parts of calcium dodecylbenzene-sulfonate, and 70 parts of xylene are blended well to give the corresponding emulsifiable concentrate.

### Formulation Example 3

Each of the present compounds a-1 to a-109, b-1 to b-43, c-1 to c-7, and d-1 to d-18 in an amount of 2 parts, 1 part of synthetic hydrous silicon oxide, 2 parts of calcium ligninsulfonate, 30 parts of bentonite, and 65 parts of kaolin clay are blended well with grinding, kneaded well with water, granulated and dried to give the corresponding granules.

### Formulation Example 4

Each of the present compounds a-1 to a-109, b-1 to b-43, c-1 to c-7, and d-1 to d-18 in an amount of 25 parts, 3 part of polyoxysorbitan monooleate, 3 parts of CMC, and 69 parts of water are blended, wet ground to a particle size of 5 µm or less to give the corresponding suspension concentrate.

### Formulation Example 5

Each of the present compounds a-1 to a-109, b-1 to b-43, c-1 to c-7, and d-1 to d-18 in an amount of 10 parts, 1 part of polyoxy-ethylenestylphenylether, and 89 parts of water are blended to give the corresponding liquid.

Next, the usefulness of the present compounds as an effective ingredient of plant disease controlling agents is illustrated in test examples. In this respect, the present compounds are represented by the compound numbers listed in Tables 3 - 4.

Also, as for the bacteriocidal or fungicidal effectiveness, the established diseases of a subject plant on examination, that is the degrees of colonies or spots on stems, leaves, and the like were visulally observed, and the severities of the plant was estimated from the ratio of spot area.

### Test Example 1 Wheat powdery mildew controlling test (treatment effect)

Sand soil was charged in a plastic bottle having a 90 ml volume, and wheat (Norin-73) was seeded and cultivated in a greenhouse for 10 days. On the young seedlings of wheat of which second leaves was developed, spore of wheat powdery mildew (Erysiphe graminis f.sp. tritici) was inoculated by spraying. After inoculation, the seedlings were grown in a growth chamber at 15°C for 2 days, an aqueous dilution of each of the formulations of the present compounds a-1, a-2, a-4, a-5, a-8, a-9 to a-15, a-18, a-20, a-23, a-29 to a-32, a-34, a-36, a-40, a-44, a-45, a-47 to a-51, a-54 to a-58, a-61 to a-63, a-65 to a-67, a-71, a-72, a-81 to a-94, a-96 to a-106, a-108, b-3, b-5 to b-8, b-10 to b-18, b-21, b-23 to b-25, b-29, b-30, b-33 to b-43, c-1 to c-7, d-3, d-4, d-7, d-9, d-11, d-13, d-14 and d-16 to d-18 prepared according to Formulation Example 2 (effective ingredient concentration 500 ppm) was sprayed so as to be attached to the surface of the young seedling of the wheat. After spraying, the seedlings were grown in a growth chamber at 15°C for 10 days to examine the attack.

As a result, while the seedlings in non-treatment area had the attack rate of 100%, the seedlings in the treated area had the attack of less than 10%.

### Test Example 2 Wheat powdery mildew controlling test (penetration transfer effect)

Sand soil was charged in a plastic bottle having a 90 ml volume, and wheat (Norin-73) was seeded and cultivated in a greenhouse for 10 days. Each of the suspension concentrations of the present compounds a-1, a-2, a-4, a-5, a-7 to a-9, a-12 to a-15, a-18, a-20 to a-23, a-29, a-36, a-39 to a-45, a-47 to a-51, a-53 to a-58, a-60 to a-63, a-65 to a-67, a-69 to a-71, a-73, a-78, a-80, a-82, a-84 to a-94, a-96 to a-105, b-3, b-5, b-8, b-10 to b-18, b-21, b-23 to b-25, b-29, b-30, b-33 to b-35, b-38, b-40 to b-43, c-1 to c-7, d-3, d-4, and d-17 prepared according to Formulation Example 4 was irrigated to the foot of the young seedlings of wheat of which second leaves were developed, so that the amount of the effective ingredient per pot reaches 2.5 mg. After treatment, the seedings were kept at room temperature (24°C) for 5 days, and the spore of wheat powdery mildew (Erysiphe graminis f.sp. tritici) was inoculated by spraying on the seedlings. After inoculation, the seedlings were kept in a growth chamber at 15°C for 10 days to examine the attack.

As a result, while the seedlings in non-treatment area had the attack of 100%, the seedlings in the area treated with the aforementioned compounds had the attack of less than 10%.

## Claims

1. A pyrazoline compound represented by the formula 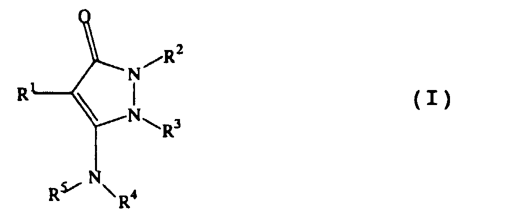 wherein R¹ represents a phenyl group which may be substituted, R² represents a hydrocarbon group which may be substituted, R³ represents an aromatic heterocyclic ring which may be substituted, R⁴ and R⁵, which may be the same or different, represent a hydrogen atom, an acyl group, a primary or secondary alkyl group which may be substituted, or are combined together to form an alkylene group which may be substituted or =CR⁶R⁷, wherein R6 represents a hydrocarbyl group which may be substituted, an alkoxy group, or a mono- or di-alkylamino group, and R⁷ represents a hydrogen atom or an alkyl group.

2. A pyrazoline compound according to Claim 1, wherein R¹ represents a phenyl group which may be substituted by one or more of the following substituents which may be the same or different, said substituents comprise a halogen atom, a C₁-C₆ alkyl group, a C₁-C₆ haloalkyl group, a C₁-C₆ alkoxy group, a C₁-C₆ haloalkoxy group, a (C₁-C₆) alkoxy (C₁-C₆) alkyl group, a C₁-C₆ alkylthio group, a C₁-C₆ haloalkylthio group, a cyano group or a nitro group, or a phenyl group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group, or a phenoxy group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group, or a C₁-C₅ alkylene group which may be substituted by the two adjacent substituents represented by -CH=CH-CH=CH-, a halogen atom or a C₁-C₃ alkyl group and forms a 3 - 7 membered cyclic ring which may contain one or two oxygen or sulfur atoms in the chain,
R² represents a C₁-C₁₀ alkyl group, a C₃-C₇ cycloalkyl group, a C₁-C₆ haloalkyl group, a (C₁-C₆) alkoxy (C₁-C₆) alkyl group, a (C₃-C₇)cycloalkyl(C₁-C₆) alkyl group, a C₃-C₁₀ alkenyl group, a C₃-C₁₀ haloalkenyl group, a (C₆-C₁₀) aryl (C₁-C₆) alkyl group, or a phenyl group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group,
R⁴ and R⁵, which may be the same or different, represents a hydrogen atom, a C₂-C₁₀ alkanoyl group, a C₂-C₁₀ haloalkanoyl group, a C₁-C₁₀ primary or secondary alkyl group, or R⁴ and R⁵ are linked together at their ends to represent a C₄-C₅ alkylene group which may be substituted by a halogen atom or a C₁-C₃ alkyl group or represent =CR⁶R⁷, wherein R⁶ represents a C₁-C₆ alkyl group, a C₁-C₆ alkoxy group, a C₁-C₆ mono- or di-alkylamino group, a phenyl group which may be substituted by a halogen atom, a C₁-C₆ alkyl group, a C₁-C₆ haloalkyl group, a C₁-C₆ alkoxy group, a C₁-C₆ haloalkoxy group, a hydroxyl group, a cyano group or a nitro group, or a pyridyl group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group, and
R⁷ represents a hydrogen atom or a C₁-C₆ alkyl group.

3. A pyrazoline compound according to Claims 1 or 2, wherein the aromatic heterocyclic ring for R³ is represented by the formula 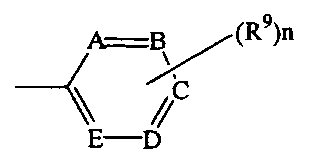 wherein R⁹ represents a C₁-C₆ alkyl group, a C₁-C₆ alkoxy group, a C₁-C₆ alkylthio group, a C₁-C₆ haloalkyl group, a C₁-C₆ haloalkoxy group, a C₁-C₆ haloalkylthio group, or a halogen atom, A, B, C, D and E, which may be the same or different, represent a CH group or a nitrogen atom, n represents an integer of 0 - 4, when either one of A - E represents a nitrogen atom, n represents an integer of 0 - 3, when either two of A - E represent a nitrogen atom, n represents an integer of 0 - 2, when either three of A - E represent a nitrogen atom, and n represents an integer of 0 - 1, when either four of A - E represent a nitrogen atom.

4. A pyrazoline compound according to Claims 2 or 3, wherein R¹ represents a phenyl group which may be substituted by the following one or more substituents which may be the same or different, said substituent(s) comprises a halogen atom, a C₁-C₆ alkyl group, a C₁-C₆ haloalkyl group, a C₁-C₆ alkoxy group, a C₁-C₆ haloalkoxy group, a C₁-C₆ alkylthio group, a cyano group or a nitro group, or a C₁-C₅ alkylene group which may be substituted by the two adjacent substituents represented by -CH=CH-CH=CH-, a halogen atom or a C₁-C₃ alkyl group and forms a 3 - 7 membered cyclic ring which may contain one or two oxygen or sulfur atoms in the chain, R⁴ and R⁵, which may be the same or different, represents a hydrogen atom, a C₂-C₁₀ alkanoyl group, a C₂-C₁₀ haloalkanoyl group, a C₁-C₁₀ primary or secondary alkyl group, or =CR⁶R⁷, wherein R⁶ represents a C₁-C₆ mono- or dialkylamino group, a phenyl group which may be substituted by a halogen atom, a C₁-C₆ alkyl group, a C₁-C₆ haloalkyl group, a C₁-C₆ alkoxy group, a C₁-C₆ haloalkoxy group, a hydroxyl group, a cyano group or a nitro group, or a pyridyl group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group.

5. A pyrazoline derivative according to Claim 3, wherein R³ represents a 2-pyridyl group, a 3-pyridyl group, a 4-pyridyl group, a 2-pyrimidinyl group, a 3-pyrimidinyl group, a 4-pyrimidinyl group, a 2-pyrazinyl group, 3-pyridazinyl group, a 4-pyridazinyl group, a 1,3,5-triazin-2-yl group, a 1,2,4-triazin-3-yl group, a 1,2,4-triazin-5-yl group, a 1,2,4-triazin-6-yl group, or a 1,2,4,5-tetrazin-3-yl group, all of which may be substituted.

6. A pyrazoline compound according to Claim 4, wherein the nitrogen-containing heterocyclic ring of the general formula 2 for R³ which may be substituted represents a 2-pyridyl group, a 2-pyrimidinyl group, and a 3-pyrimidinyl group.

7. A plant disease controlling agent, which comprises the pyrazoline compound according to Claim 1 as an effective ingredient.

8. A process for controlling plant diseases, which comprises applying the pyrazoline compound according to Claim 1 to a plant.

9. An aminopyrazoline compound represented by the formula (II): 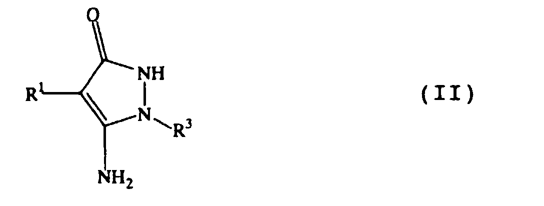 wherein R¹ represents a phenyl group which may be substituted, and R³ represents an aromatic heterocyclic ring which may be substituted.

10. An aminopyrazoline compound according to Claim 9, wherein R¹ represents a phenyl group which may be substituted by the following one or more substituents which may be the same or different, said substituent comprises a halogen atom, a C₁-C₆ alkyl group, a C₁-C₆ haloalkyl group, a C₁-C₆ alkoxy group, a C₁-C₆ haloalkoxy group, a (C₁-C₆) alkoxy (C₁-C₆) alkyl group, a C₁-C₆ alkylthio group, a C₁-C₆ haloalkylthio group, a cyano group or a nitro group, or a phenyl group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group, or a phenoxy group which may be substituted by a halogen atom, a C₁-C₆ alkyl group, a C₁-C₆ haloalkyl group, a C₁-C₆ alkoxy group, a C₁-C₆ haloalkoxy group, a cyano group or a nitro group, or a C₁-C₅ alkylene group which may be substituted by the two adjacent substituents represented by -CH=CH-CH=CH-, a halogen atom or a C₁-C₃ alkyl group and forms a 3 - 7 membered cyclic ring which may contain one or two oxygen or sulfur atoms in the chain.

11. An aminopyrazoline compound according to Claim 9 or 10, wherein the aromatic heterocyclic ring for R³ is represented by the formula 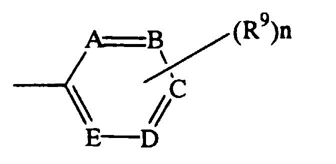 wherein R⁹ represents a C₁-C₆ alkyl group, a C₁-C₆ alkoxy group, a C₁-C₆ alkylthio group, a C₁-C₆ haloalkyl group, a C₁-C₆ haloalkoxy group, a C₁-C₆ haloalkylthio group, or a halogen atom, A, B, C, D and E, which may be the same or different, represent a CH group or a nitrogen atom, n represents an integer of 0 - 4, when either one of A - E represents a nitrogen atom, n represents an integer of 0 - 3, when either two of A - E represent a nitrogen atom, n represents an integer of 0 - 2, when either three of A - E represent a nitrogen atom, and n represents an integer of 0 - 1, when either four of A - E represent a nitrogen atom.

12. An aminopyrazoline compound according to Claim 10 or 11, wherein R¹ represents a phenyl group which may be substituted by one or more of the following substituents which may be the same or different, said substituents comprise a halogen atom, a C₁-C₆ alkyl group, a C₁-C₆ haloalkyl group, a C₁-C₆ alkoxy group, a C₁-C₆ haloalkoxy group, a C₁-C₆ alkylthio group, a cyano group or a nitro group, or a C₁-C₅ alkylene group which may be substituted by the two adjacent substituents represented by -CH=CH-CH=CH-, a halogen atom or a C₁-C₃ alkyl group and forms a 3 - 7 membered cyclic ring which may contain one or two oxygen or sulfur atoms in the chain.

13. An aminopyrazoline compound according to Claim 11, wherein R³ represents a 2-pyridyl group, a 3-pyridyl group, a 4-pyridyl group, a 2-pyrimidinyl group, a 3-pyrimidinyl group, a 4-pyrimidinyl group, a 2-pyrazinyl group, 3-pyridazinyl group, a 4-pyridazinyl group, a 1,3,5-triazin-2-yl group, a 1,2,4-triazin-3-yl group, a 1,2,4-triazin-5-yl group, a 1,2,4-triazin-6-yl group, or a 1,2,4,5-tetrazin-3-yl group, all of which may be substituted.

14. A pyrazoline compound according to Claim 11, wherein R³ represents a 2-pyridyl group, a 2-pyrimidinyl group, or a 3-pyrimidinyl group, all of which may be substituted.
